(19) Europäisches Patentamt
European Patent Office
Office européen des brevets

(11) EP 4 450 649 B1

(12) **EUROPEAN PATENT SPECIFICATION**

(45) Date of publication and mention of the grant of the patent:
**19.11.2025 Bulletin 2025/47**

(21) Application number: **24153686.1**

(22) Date of filing: **24.01.2024**

(51) International Patent Classification (IPC):
***C12Q 1/689*** *(2018.01)* ***G16H 50/30*** *(2018.01)*
***G16H 50/20*** *(2018.01)*

(52) Cooperative Patent Classification (CPC):
**C12Q 1/689; G16H 50/20; G16H 50/30;**
C12Q 2600/112

(54) **METHOD AND SYSTEM FOR RISK ASSESSMENT OF AUTISM SPECTRUM DISORDER IN A SUBJECT**

VERFAHREN UND SYSTEM ZUR RISIKOBEURTEILUNG VON STÖRUNGEN DES AUTISMUSSPEKTRUMS BEI EINER PERSON

PROCÉDÉ ET SYSTÈME D'ÉVALUATION DE RISQUE DE TROUBLE DU SPECTRE AUTISTIQUE CHEZ UN SUJET

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **19.04.2023 IN 202321028609**

(43) Date of publication of application:
**23.10.2024 Bulletin 2024/43**

(73) Proprietor: **Tata Consultancy Services Limited**
**Maharashtra (IN)**

(72) Inventors:
- **NAGPAL, SUNIL**
**411013 Pune, Maharashtra (IN)**
- **HAQUE, MOHAMMED MONZOORUL**
**411013 Pune, Maharashtra (IN)**
- **MANDE, SHARMILA SHEKHAR**
**411013 Pune, Maharashtra (IN)**
- **MERCHANT, MITALI**
**411013 Pune, Maharashtra (IN)**
- **CHENNAREDDY, VENKATA SIVA KUMAR REDDY**
**500001 Hyderabad, Telangana (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**WO-A1-2020/086967**

- **QIAO YANAN ET AL: "Alterations of oral microbiota distinguish children with autism spectrum disorders from healthy controls", vol. 8, no. 1, 25 January 2018 (2018-01-25), US, XP093177466, ISSN: 2045-2322, Retrieved from the Internet <URL:https://www.nature.com/articles/s41598-018-19982-y.pdf> DOI: 10.1038/s41598-018-19982-y**
- **KONG XUEJUN ET AL: "New and Preliminary Evidence on Altered Oral and Gut Microbiota in Individuals with Autism Spectrum Disorder (ASD): Implications for ASD Diagnosis and Subtyping Based on Microbial Biomarkers", NUTRIENTS, vol. 11, no. 9, 6 September 2019 (2019-09-06), CH, pages 2128, XP093177557, ISSN: 2072-6643, DOI: 10.3390/nu11092128**
- **OLSEN INGAR ET AL: "Oral microbiota and autism spectrum disorder (ASD)", vol. 12, no. 1, 14 August 2018 (2018-08-14), SE, pages 1702806, XP093177564, ISSN: 2000-2297, Retrieved from the Internet <URL:https://www.ncbi.nlm.nih.gov/pmc/articles/PMC6913665/pdf/ZJOM_12_1702806.pdf> DOI: 10.1080/20002297.2019.1702806**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

CROSS-REFERENCE TO RELATED APPLICATIONS AND PRIORITY

The present application claims priority to Indian application no. 202321028609, filed on April 19, 2023.

TECHNICAL FIELD

The disclosure herein generally relates to a risk assessment of disorders present in a subject and, more particularly, to method and system for a risk assessment of autism spectrum disorder (ASD) present in the subject and designing a personalized recommendation for the same.

BACKGROUND

Autism spectrum disorder (ASD) refers to a spectrum of serious neurodevelopmental or pervasive developmental disorders especially in children that impair their social, communicative, linguistic, cognitive, and behavioral traits. With a worldwide (mean) prevalence of 1 in every 160 children (WHO, 2017), and more than 3.5 million identified cases in US alone, methods for assessing and treating this disorder has long eluded the scientific community. The ASD affected children seek and require significantly higher care and expenses, which include regular monitoring of the condition, routine therapy, special education, and associated healthcare expenses. As per a report on the economic costs of ASD, US spent a total of approximately $11.5 billion in 2011.

Currently available ASD related diagnostic tools and procedures, though abundant in numbers, are predominantly based on psychiatric or behavioral evaluations, checklists and associated statistical inferences, which highlight the inherent limitation in making a reliable and early diagnosis. Several therapeutic regimens have also been attempted in the last few decades, including from use of psychedelic drugs like LSD (1960s), electric shock treatments (in 1970s) to the administration of FDA approved antipsychotic medicines like risperidone, which have focused more (rather completely) on temporary amelioration of ASD related behavior(s) than the cure. Although recent literature exists pertaining to oral microbiome as a possible avenue towards diagnosis and treatment of ASD, it mainly represents a subjective approach and is primarily aimed at comparative analysis of controls and subjects having ASD. Hence, the conventional techniques and procedures for risk assessment and treatment of ASD are not efficient and temporary in nature.

QIAO YANAN ET AL: "Alterations of oral microbiota distinguish children with autism spectrum disorders from healthy controls" discloses altered gut microbiota is associated with autism spectrum disorders (ASD), a group of complex, fast growing but difficult-to-diagnose neurodevelopmental disorders worldwide. However, the role of the oral microbiota in ASD remains unexplored. Via high-throughput sequencing of 111 oral samples in 32 children with ASD and 27 healthy controls, we demonstrated that the salivary and dental microbiota of ASD patients were highly distinct from those of healthy individuals. Lower bacterial diversity was observed in ASD children compared to controls, especially in dental samples. Also, principal coordinate analysis revealed divergences between ASD patients and controls. Moreover, pathogens such as Haemophilus in saliva and Streptococcus in plaques showed significantly higher abundance in ASD patients, whereas commensals such as Prevotella, Selenomonas, Actinomyces, Porphyromonas, and Fusobacterium were reduced. Specifically, an overt depletion of Prevotellaceae co-occurrence network in ASD patients was obtained in dental plaques. The distinguishable bacteria were also correlated with clinical indices, reflecting disease severity and the oral health status (i.e. dental caries). Finally, diagnostic models based on key microbes were constructed, with 96.3% accuracy in saliva. Taken together, this study characterized the habitat-specific profile of the oral microbiota in ASD patients, which might help develop novel strategies for the diagnosis of ASD (Abstract).

SUMMARY

Embodiments of the present disclosure present technological improvements as solutions to one or more of the above-mentioned technical problems recognized by the inventors in conventional systems.

In an aspect, a method for risk assessment of autism spectrum disorder in a subject is provided. The invention is set out in the appended set of claims 1-7.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for risk assessment of autism spectrum disorder present in the subject, according to some embodiments of the present disclosure.

FIGS. 2A and 2B are flowcharts illustrating a method for risk assessment of autism spectrum disorder present in the subject, according to some embodiments of the present disclosure.

FIG. 3A illustrates an exemplary probe and multiplexed qPCR design for detecting and determining the quantitative abundance of each of a plurality of predetermined microbes associated with a saliva sample, according to some embodiments of the present disclosure.

FIG. 3B illustrates an exemplary probe and multiplexed qPCR design for detecting and determining the" quantitative abundance of each of the plurality of predetermined microbes associated with a dental plaque sample, according to some embodiments of the present disclosure.

FIGS. 4A, 4B and 4C are flowcharts illustrating steps involved in building a pre-determined machine learning model according to some embodiments of the present disclosure.

FIG. 5 illustrates an exemplary block diagram of a kit for risk assessment of autism spectrum disorder present in the subject, according to some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Role of brain's cerebral cortex in deciding the intelligence, behavior, personality, perception, and language processing capabilities of an individual is very well established. Several studies have rather attempted to understand the changes in cerebral cortex of autism spectrum disorder (ASD) affected subjects as well. Given the fact that oral tissue has dense sensory innervations connecting the oral environment to the cerebral cortex, the hypothesis that a change in oral microbiota and metabolites thereof could have profound impact on functioning of cerebral cortex, begs due attention.

Last 15 years have also seen a huge surge in interest towards understanding the role of human microbiome in various disease states. Gut microbiome in particular has received a lot of attention and innumerable studies have established firm scientific evidence linking the changes in gut microbiota with onset, progression and/or even reversal of disease states. The theory of gut-brain axis, focusing on the duplex communication between the central and the enteric nervous system, and a consequent connection of emotional and cognitive centers of brain with the functioning of intestine(s) particularly got an intriguing turn when the idea of the effects of gut microbiota on intestinal function (and hence the nervous function) is disclosed. Ever since, multitude of scientific groups have explored this paradigm of the effects of gut microbes on brain behavior, especially in the context of neurodevelopmental and neurodegenerative disorders. Alterations in the gut microbial community structure of ASD subjects have consistently been reported in the recent past. There are conventional techniques emerged that make use of specific *gut microbial* species as prospective therapeutic or diagnostic markers for ASD. A reliable translatable product is yet to reach the market though.

Some of the existing diagnostic and therapeutic methods, and their limitations are summarized below:

(i) Psychiatric or behavioral evaluations, checklists and associated statistical inferences, even though constitute the prevailing and accepted state of art in diagnosis, they merely represent qualitative diagnosis based on human judgments. No physical trait or behavior can be the sole and unique representative of the actual physiological state of mind. Even the diagnostic evaluations and checklists themselves make a declaration that the outcomes of the tests are not conclusive evidence of the ASD.

(ii) Diagnostic tests for the ASD are not available or very limited to the above-mentioned evaluative checklists.

(iii) Microbiome as a biomarker for the ASD has been attempted, but most of them are confined to gut microbiome with limited accuracies.

(iv) Oral microbiome as a biomarker for the ASD is present, but mainly represents a subjective approach aimed at comparative analysis of controls and ASD subjects. A random forest-based approach for classification of samples relies on as high as 36 Operational Taxonomic Units (OTUs) or microbes in saliva samples alone and as high as 54 OTUs or microbes in plaque samples alone. Dependence on a large number of microbial features for developing a diagnostic test is neither easily translatable nor economical. Moreover, it is difficult to arrive at a relevant and relatable therapeutic solution based on such a large number of microbes in a diagnostic marker.

(v) Several therapeutic regimes have been attempted in the last (more than) 100 years of ASD's history, ranging from use of psychedelic drugs like LSD (1960s), electric shock treatments (in 1970s) to the administration of FDA approved anti-psychotic medicines like risperidone, which have focused more (rather completely) on temporary amelioration of behavior(s) than the cure. In the current state of art, no therapeutic drug or supplement exists for curing the ASD.

The present disclosure solves the challenges present in the existing state of art, for accurate identification and selection of subjects with increased risk of the ASD and providing a personalized microbial cocktail for treating the ASD, using oral microbiome of the ASD affected subjects. The present disclosure attempts to offer an early, easy and reliable risk assessment and a method for design of personalized recommendable composition for applications towards amelioration of ASD.

The present disclosure involves a paired extraction and quantification of site-specific unique microbial sequences (pertaining to the oral microbial samples) of an ASD subject and subsequent classification of the subject under the ASD risk category using a metric based on a predefined ensemble of mathematical formulas (using ensemble machine learning approach) specific to some unique (not all) combinations of extracted microbial sequences. Each formula in the entire ensemble contains a mix of microbial sequences from the microbial sample sites. Further, a guided development of personalized recommended microbial cocktail (s) is then carried out based on the most relevant formula-set for the subject. The identification of the formula ensemble, its constituting combination of microbial sequences, and associated mathematical relationships and constants/coefficients, is dependent on an independently devised ensemble machine learning approach.

Referring now to the drawings, and more particularly to FIG. 1 through 5, where similar reference characters denote corresponding features consistently throughout the figures, there are shown preferred embodiments and these embodiments are described in the context of the following exemplary system and/or method.

FIG. 1 illustrates an exemplary block diagram of a system 100 for risk assessment of autism spectrum disorder present in the subject, according to some embodiments of the present disclosure. In an embodiment, the system 100 includes a memory 102, a database 104, one or more hardware processors 106, a sample collection module 108, a DNA extraction module 110, an abundance determining module 112, a machine learning (ML) module 114, an assessment module 116, and a recommendation module 118. In an embodiment, the database 104 and the machine learning (ML) module 114 are stored in the memory 102.

In an embodiment, the sample collection module 108 is configured to collect a saliva sample and a dental plaque sample of the subject whose risk of autism spectrum disorder is to be assessed. The DNA extraction module 110 is configured to extract microbial deoxyribonucleic acid (DNA) sequences from each of the saliva sample and the dental plaque sample, individually. The abundance determining module 112 is configured to determine a quantitative abundance of: (i) each of a plurality of predetermined microbes associated with the saliva sample and (ii) each of a plurality of predetermined microbes associated with the dental plaque sample, individually, from respective extracted DNA sequences.

The machine learning (ML) module 114 is configured to determine a model score based on the hybrid abundance matrix which is obtained by collating the quantitative abundance of: (i) each of the plurality of predetermined microbes associated with the saliva sample and (ii) each of the plurality of predetermined microbes associated with the dental plaque sample. The assessment module 116 is configured to perform risk assessment of the subject, based on the model score. Lastly, the recommendation module 118 is configured to design a personalized recommendation for the subject assessed as having the autism spectrum disorder.

In an embodiment, the one or more hardware processors 106 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 106 is configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, a network cloud and the like.

The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes.

Further, the memory 102 may include a database 104 configured to include information regarding risk assessment of autism spectrum disorder present in the subject. The memory 102 may comprise information pertaining to input(s)/output(s) of each step performed by the one or more hardware processors 106 of the system 100 and methods of the present disclosure. In an embodiment, the database 104 may be external (not shown) to the system 100 and coupled to the system 100 via the I/O interfaces (not shown in FIG. 1).

In an embodiment, one or more data storage devices or the memory 102 operatively coupled to the one or more hardware processors 106. The system 100 with the one or more hardware processors 106 is configured to execute functions of one or more functional modules of the system 100.

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail.

In an embodiment, the memory 102 comprises one or more data storage devices operatively coupled to the one or more hardware processors 106 and is configured to store instructions for execution of steps of the method depicted in FIGS. 2A and 2B by the one or more hardware processors 106. FIGS. 2A and 2B are flowcharts illustrating a method 200 for risk assessment of autism spectrum disorder present in the subject, according to some embodiments of the present disclosure. The steps of the method 200 of the present disclosure will now be explained with reference to the components or blocks of the system 100 as depicted in FIG. 1 and the steps of flow diagrams as depicted in FIGS. 2A and 2B. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of the method 200, the saliva sample and the dental plaque sample of the subject whose risk of the autism spectrum disorder (ASD) is to be assessed, are collected through the sample collection module 108. In an embodiment, the subject or the individual is a human being. Both the saliva sample and the dental plaque sample are collected (together) as a pair at the same time, from the same subject whose risk of the ASD is to be assessed. As the ASD is very important to be assessed especially in children, the subject preferably is a human child. However, the adoption of similar sampling, assessment and recommendation strategy in other age groups is well within the scope of the present disclosure.

Both the saliva sample and the dental plaque sample are oral microbial and site-specific samples and are non-invasive. In an embodiment, the saliva sample may refer to extracted salivary swabs or naturally out-flown saliva or voluntarily spitted saliva which is obtained in a non-stimulatory environment (where stimulations refer to behavioral or digestive triggers). In general, the saliva sample is extracted from the mouth site of the subject. The dental plaque samples refer to the scraps obtained from oral site-specific area of the subject where the teeth are present. In an embodiment, the scraps can be obtained using instruments such as curettes.

Further, at step 204 of the method 200, microbial deoxyribonucleic acid (DNA) sequences from each of the saliva sample and the dental plaque sample collected at step 202 of the method 200, are extracted individually through the DNA extraction module 110. In an embodiment, the extraction of microbial DNA sequences from both the saliva sample and the dental plaque sample is performed by amplification of 16S rRNA marker genes (either full-length or specific variable regions of the gene) using one or more of: a next-generation sequencing (NGS) platform, Oxford nanopore sequencing or any other DNA sequencing technique and platform (including a classical Sanger sequencing). In another embodiment, the NGS platforms include any one of whole genome sequencing, CPN60 gene-based amplicon sequencing, other phylogenetically conserved genetic region-based amplicon sequencing, sequencing using approaches which involve either a fragment library or a mate-pair library or a paired-end library or a combination of the same. Further, the DNA extraction module 110 includes taxonomic classification of the sequenced reads at genus level using RDP, and latest version of any other taxonomic classification database such as Greengenes or Silva databases, or algorithms such as dada2 are covered in the scope of this invention

Further, at step 206 of the method 200, the quantitative abundance of:

(i) each of a plurality of predetermined microbes associated with the saliva sample and
(ii) each of a plurality of predetermined microbes associated with the dental plaque sample, is determined individually, from respective extracted DNA sequences extracted at step 204 of the method 200. More specifically, the quantitative abundance of each of the plurality of predetermined microbes associated with the saliva sample is determined from the microbial DNA sequences corresponding to the saliva sample. Similarly, the quantitative abundance of each of the plurality of predetermined microbes associated with the dental plaque sample is determined from the microbial DNA sequences corresponding to the dental plaque sample. The plurality of predetermined microbes is the corresponding operational taxonomy units (OTUs) whose quantification is performed in the corresponding sample.

In an embodiment, the plurality of predetermined microbes associated with the saliva sample are: *Mogibacterium, Peptostreptococcus, Eubacterium, Solobacterium, Actinomyces,* and *Alistipes.* In an embodiment, the plurality of predetermined microbes associated with the dental plaque sample are: *Eubacterium, Dialister, Atopobium, Enterococcus, Mogibacterium,* and *Anaeroglobus.* The scope of the present disclosure is not limited to the mentioned names of the predetermined microbes and applicable to other alternative names of the microbes present in the art.

The marker feature sequences of some of the predetermined microbes are listed below:

> Mogibacterium:

1 gatgaacgct ggcggcgtgc ctaatacatg caagtcgagc gagaagcttg gaaatgacgc
61 ttcggttgaa tttccaagcg gacagcggcg gacgggtgag taacgcgtag gcaacctgcc
121 cctgtacaga gggatagcca ttggaaacga tgattaaaac ctcatgacac cgtagtagca
181 catgctacat cggtcaaaga tttatcggtc agggatgggc ctccgtctga ttaactggtt
241 ggtgaggtaa cggctcacca aggtgacgat cagtagccga cctgagaggg tgatcggcca
301 cattggaact gagacacggt ccaaacttct acggaaggca gcagtaggga atcttgcaca
361 atgggcgtaa gcctgatgca gcaacgccgc gtgaaggatg aaggcttcgg gttgtaaact
421 tctgttctaa gggaagaaag aaatgacggt accttaggag caagccccgg ctaactacgt
481 gccagcagcc gcggtaatac gtagggggca agcgttatcc ggaattattg ggcgtaaaga
541 gtgcgtaggt ggttacctaa gcgcaaggtt taaattagag gctcaacctc tacatgcctt
601 gcgaactggg ctacttgagt gcaggagggg aaagcggaat tcctagtgta gcggtgaaat
661 gcgtagatat taggaggaac accggcggcg aaggcggctt tctggactgt aactgacact
721 gaggcacgaa agcgtgggta gcaaacagga ttagataccc tggtagtcca cgccgtaaac
781 gatgagcact aggtgttggg tccgttagga ctcagtgccg cagttaacgc aataagtgtc
841 cgcctgggag tacgctcgca agagtaaact caaggaattg acgggcaccc gcacaagcag
901 gggagcttgt ggtttaattc gaagcaacgc gaagaacctt accagggctt gacatcctgc
961 tgacagaacc ttaatcggtt ttttcttcgg acagcagaga caggtggtgc atggttgtcg
1021 tcagctcgtg tcgtgagatg ttgggttaag tcccgcaacg agcgcaaccc ttgtcgctag
1081 ttactaacat tcagttgagg actctagcga gactgccgag gtcaactcgg aggaaggtgg
1141 ggatgacgtc aaatcatcat gccccttatg ttctgggcta cacacgtgct acaatggtcg
1201 gtacaatgag atgcaatact gcgaagtgga gcgaaacacc aaaaccgatc ccagttcgga
1261 ttgtaggctg caactcgcct acatgaagtc ggagttgcta gtaatcgcag atcagaatgc
1321 tgcggtgaat gcgttcccgg gtcttgtaca caccgcccgt cacaccatgg aagttggggg
1381 tgcccaaagt cggttaatta atctatcgcc taaggcaaaa ccaatgactg gggt

> Eubacterium:

1 gtgctgcaga gaagagtttg atcctggctc aggatgaacg ctggcggcgt gcctaacaca
61 tgcaagttga gcgagaaatc acttaaagaa gcttcggtag acttaagaga tggagagcag
121 cggacgggtg aggaacgcgt gggaaacctg cccttgacag gaggatagcc gagagaaatt
181 tcgatttaat acttcataaa gcagagcatt cgcatggatg aactgccaaa gaattatcgg
241 tcaaggatgg tccagcgtct gattagctgg ttggtaaggt accggcttac caaggcgacg
301 atcagtagcc ggcctgagag ggtgaacggc cacattggaa ctgagacacg gtccagactc
361 tacgggaggc agcagtgggg aatattgcac aatgggcgaa gcctgatgca gcaacgccgc
421 gtgaaggaag aaggctttcg agtcgtaaac ttctgtccaa agggaagaat aatgacggta
481 cctttgaaga aagccccggc taactacgtg ccagcagccg cggtaatacg tagggggcga
541 gcgttatccg gaattactgg gcgtaaagag tatgtaggtg gttaagtaag cgtagggttt
601 aaggcgacag cccaactgtc gtatgccccg cgagctgttt aacttgagta caggaggga
661 aggcggaatt cctagtgtag cgctgaaatg cgtagatatt aggaggaaca ccagtggcga
721 aggcggcctt ctggactgta actgacactg agatacgaaa gcgtgggtag caaacaggat
781 tagataccct ggtagtccac gccgtaaacg atgagcacta ggtgtcgggc tcgcaagagt
841 tcggtgccgg agcaaacgca ttaagtgctc cgcctgggga gtacgcacgc aagtgtgaaa
901 ctcaaaggaa ttgacgggga cccgcacaag cagcggagca tgtggtttaa ttcgaagcaa
961 cgcgaagaac cttaccagga cttgacatcc cactgaaagc tcggttaaaa ctgagccctt
1021 cttcggaaca gtggagacag gtggtgcatg gttgtcgtca gctcgtgtcg tgagatgttg
1081 ggttaagtcc cgcaacgagc gcaacccttg ccgttagttg ccatcattaa gttgggcact
1141 ctaatgggac tgccggggag aacccggagg aaggcgggga tgacgtcaaa tcatcatgcc
1201 ccttatgttc tgggctacac acgtgctaca atggccgtca cagagggaag cgagagagcg
1261 atcttaagcg aaaccaaaaa ggcggtccca gttcggactg caggctgcaa ctcgcctgca
1321 cgaagccgga gttgctagta atcgcggatc agaatgtcgc ggtgaatgcg ttcccgggtc
1381 ttgtacacac cgcccggtgga tccgtg

> Dialister:

1 cgaacgctgg cggcgtgctt aacacatgca agtcgaacgg aaagagatga aagagcttgc

61 tcttttatta atttcagtgg caaacgggtg agtaacacgt aaacaacctg ccttaaggat
121 ggggataaca gacggaaacg actgctaata ccgaatacgt tctaagcatc gcatggtgca
181 tagaagaaag ggtggcctct acaagaaagc tatcgcctta agaggggttt gcgactgatt
241 aggtagttgg tgaggtaacg gctcaccaag ccgacgatca gtagccggtc tgagaggatg
301 aacggccaca ctggaactga gacacggtcc agactcctac gggaggcagc agtggggaat
361 cttccgcaat ggacgaaagt ctgacggagc aacgccgcgt gaacgaagaa ggtcttcgga
421 ttgtaaagtt ctgtgattcg ggacgaaagg gtttgtggtg aataatcatt gacattgacg
481 gtaccgaaaa agcaagccac ggctaactac gtgccagcag ccgcggtaat acgtaggtgg
541 caagcgttgt ccggaattat tgggcgtaaa gcgcgcgcag gcggtttctt aagtccatct
601 taaaagcgtg gggctcaacc ccatgagggg atggaaactg ggaagctgga gtatcggaga
661 ggaaagtgga attcctagtg tagcggtgaa atgcgtagag attaggaaga acaccggtgg
721 cgaaggcgac tttctagacg aaaactgacg ctgaggcgcg aaagcgtggg gagcaaacag
781 gattagatac cctggtagtc cacgccgtaa acgatggata ctaggtgtag gaggtatcga
841 cccctcctgt gccggagtta acgcaataag tatcccgcct gggaagtacg atcgcaagat
901 taaaactcaa aggaattgac gggggcccgc acaagcggtg gagtatgtgg tttaattcga
961 cgcaacgcga agaaccttac caagccttga cattgatcgc aatccataga aatatggagt
1021 tcttcttcgg aagacgagaa aacaggtggt gcacggctgt cgtcagctcg tgtcgtgaga
1081 tgttgggtta agtcccgcaa cgagcgcaac ccctatttcc tgttaccagc acgtaaaggt
1141 ggggactcag gagagaccgc cgcggacaac gcggaggaag gcggggatga cgtcaagtca
1201 tcatgcccct tatggcttgg gctacacacg tactacaatg ggtgccaaca aagagaagcg
1261 aaatcgcgag atggagcgga cctcataaac gcacccccag ttcagattgc aggctgcaac
1321 tcgcctgcat gaagtaggaa tcgctagtaa tcgcgggtca gcataccgcg gtgaatacgt
1381 tcccgggcct tgtacacacc gcccgtcaca ctatgagagt tagagacacc cgaagccggt
1441 gaggtaaccg caagggacca gccgtctaag gtggagctga tgattggagt gaagtcgtaa
1501 caag

> Enterococcus:

1 agagtttgat cctggctcag gacgaacgct ggcggcgtgc ctaatacatg caagtcgaac

61 gcttctttcc tcccgagtgc ttgcactcaa ttggaaagag gagtggcgga cgggtgagta
121 acacgtgggt aacctaccca tcagaggggg ataacacttg gaaacaggtg ctaataccgc
181 ataacagttt atgccgcatg gcataagagt gaaaggcgct ttcgggtgtc gctgatggat
241 ggacccgcgg tgcattagct agttggtgag gtaacggctc accaaggcca cgatgcatag
301 ccgacctgag agggtgatcg gccacactgg gactgagaca cggcccagac tctacgggag
361 gcagcagtag ggaatcttcg gcaatggacg aaagtctgac cgagcaacgc cgcgtgagtg
421 aagaaggttt tcggatcgta aaactctgtt gttagagaag aacaaggacg ttagtaactg
481 aacgtcccct gacggtatct aaccagaaag ccacggctaa ctacgtgcca gcagccgcgg
541 taatacgtag gtggcaagcg ttgtccggat ttattgggcg taaagcgagc gcaggcggtt
601 tcttaagtct gatgtgaaag cccccggctc aaccggggag ggtcattgga aactgggaga
661 cttgagtgca gaagaggaga gtggaattcc atgtgtagcg gtgaaatgcg tagatatatg
721 gaggaacacc agtggcgaag gcggctctct ggtctgtaac tgacgctgag gctcgaaagc
781 gtggggagca aacaggatta gataccctgg tagtccacgc cgtaaacgat gagtgctaag
841 tgttggaggg tttccgccct tcagtgctgc agcaaacgca ttaagcactc cgcctgggga
901 gtacgaccgc aaggttgaaa ctcaaaggaa ttgacggggg cccgacaagc ggtggagcat
961 gtgtttaatt cgaagcaacg cgaagaacct taccaggtct tgacatcctt tgaccactct
1021 agagatagag ctttcccttc ggggacaaag tgacagtggt gcatggttgt cgtcagctcg
1081 tgtcgtgaga tgttgggtta agtccgcaac gagcgcaacc cttattgtta gttgccatca
1141 tttagttggg cactctagcg agactgccgg tgacaaaccg gaggaaggtg gggatgacgt
1201 caaatcatca tgccccttat gacctgggct acacacgtgc tacaatggga agtacaacga
1261 gtcgctagac cgcgaggtca tgcaaatctc ttaaagcttc tctcagttcg gattgcaggc
1321 tgcaactcgc ctgcatgaag ccggaatcgc tagtaatcgc ggatcagcac gccgcggtga
1381 atacgttccc gggccttgta cacaccgccc gtcacaccac gagagtttgt aacacccgaa
1441 gtcggtgagg taaccttttt ggagccagcc gcctaaggtg ggatagatga tttgggtgaa
1501 gtcgtaacaa ggtaacc

> Anaeroglobus:

1 cttacgatcg agtggcaaac gggtgagtaa cgcgtaaaca acctgccccg cagatgggga

61 caacagctgg aaacggctgc taataccgaa tacggtcctc ttagcgcatg gtaagaggaa
121 gaaagggtgg cctctggaac aagctaccgc tgtgggaggg gtttgcgtct gattagctgg
181 ttggaggggt aacggcccac caaggcgacg atcagtagcc ggtctgagag gatgaacggc
241 cacattggaa ctgagacacg gtccagactc ctacgggagg cagcagtggg gaatcttccg
301 caatgggcga aagcctgacg gagcaacgcc gcgtgagtga agacggcctt cgggttgtaa
361 agctctgtta taggggacga acggccgggt agcgaagagg tagccggcat gacggtaccg
421 taagagaaag ccacggctaa ctacgtgcca gcagccgcgg taatacgtag gtggcaagcg
481 ttgtccggaa tgattgggcg taaagggcgc gcaggcggct gtgtaagtct gtctaaaaag
541 tgcggggcta aaccccgtga gaggatggaa actggacagc tgagagtgtc ggagaggaaa
601 gcggaattcc tagtgtagcg gtgaaatgcg tagatattag gaggaacacc ggtggcgaaa
661 gcggctttct ggacgacaac tgacgctgag gcgcgaaagc caggggagca aacgggatta
721 gataccccgg tagtcctggc cgtaaacgat gggtactagg tgtaggaggt atcgacccct
781 cctgtgccgg agttaacgca ataagtaccc cgcctgggga gtacggccgc aaggctgaaa
841 ctcaaaggaa ttgacggggg cccgcacaag cggtggagta tgtggtttaa ttcgacgcaa
901 cgcgaagaac cttaccaagc cttgacattg ctcgcaacgg gtagagatac ctggttcttc
961 ttcggaagac gagacaacag gtggtgcacg gctgtcgtca gctcgtgtcg tgagatgttg
1021 ggttaagtcc cgcaacgagc gcaaccccta tcttcagtta ccagcacgta gcggtgggga
1081 ctcaggagag acagccgcag acaatgcgga ggaaggcggg gacgacgtca agtcatcatg
1141 ccccttatgg cttgggctac acacgtacta caatggctct aaatagaggg aagcgaagga
1201 gcgatccgga gcaaaccccg caaacagagt cccagttcgg attgcaggct gcaactcgcc
1261 tgcatgaagg aggaatcgct agtaatcgca ggtcagcata ctgcggtgaa tacgttcccg
1321 ggccttgtac acaccgcccg tcacaccacg aaagtcattc acacccgaag ccggtgaggt
1381 aaccgcaagg agccagccgt caaaggtg

> Peptostreptococcus:

1 gatgaacgct ggcggcgtgc ctaacacatg caagtcgagc gagggtttgc tcagtattga
61 gtattctaag actagaatgt tcaattctga gcaaaaccaa gcggcggacg ggtgagtaac
121 gcgtgggtaa cctgccctat acacatggat aacatactga aaagtttact aatacatgat

181 aatatatatt tgcggcatcg cagatatatc aaagtgttag cggtatagga tggacccgcg
241 tctgattagc tagttggtga gataactgcc caccaaggcg acgatcagta gccgacctga
301 gagggtgatc ggccacattg gaactgagac acggtccaaa ctcctacggg aggcagcagt
361 ggggaatatt gcacaatggg cgaaagcctg atgcagcaac gccgcgtgaa cgatgaaggt
421 cttcggatcg taaagttctg ttgcagggga agataatgac ggtaccctgt gaggaagccc
481 cggctaacta cgtgccagca gccgcggtaa tacgtagggg gctagcgtta tccggattta
541 ctgggcgtaa agggtgcgta ggtggtcctt caagtcggtg gttaaaggct acggctcaac
601 cgtagtaagc cgccgaaact ggaggacttg agtgcaggag aggaaagtgg aattcccagt
661 gtagcggtga aatgcgtaga tattgggagg aacaccagta gcgaaggcgg ctttctggac
721 tgcaactgac actgaggcac gaaagcgtgg gtagcaaaca ggattagata ccctggtagt
781 ccacgctgta aacgatgagt actaggtgtc gggggttacc cccctcggtg ccgcagctaa
841 cgcattaagt actccgcctg gggagtacgc acgcaagtgt gaaactcaaa ggaattgacg
901 gggacccgca caagtagcgg agcatgtggt ttaattcgaa gcaacgcgaa gaaccttacc
961 taagcttgac atccctcgga caggtgttta atcacaccct tccttcggga ctgaggtgac
1021 aggtggtgca tggttgtcgt cagctcgtgt cgtgagatgt tgggttaagt cccgcaacga
1081 gcgcaaccct tgtctttagt tgccagcatt cagttgggca ctctagagag actgccaggg
1141 ataacctgga ggaaggtggg gatgacgtca aatcatcatg ccccttatgc ttagggctac
1201 acacgtgcta caatgggtgg tacagagggt tgccaaaccg tgaggtggag ccaatccctt
1261 aaagccattc tcagttcgga ttgtaggctg aaactcgcct acatgaagct ggagttacta
1321 gtaatcgcag atcagaatgc tgcggtgaat gcgttcccgg gtcttgtaca caccgcccgt
1381 cacaccacgg gagtcggaaa cacccgaagc cgattatcca accgcaagga ggaagtcgtc
1441 gaaggtggcg tcnataactg gggtg

> Solobacterium:

1 agagtttgat cctggctcag tatgaactct gccggcgtgc ctaatacttg caagtcgaac
61 gctgaagatc tagcttgcta gatcgaagga gtgccgaacg ggtgagtaat acataagcaa
121 cctacccacg aagactggga taatctctgg aaacggggac taataccgga taggtaatcg
181 gaaggcatct tctggttatt aaaggttaaa aacactggtg gatgggctta tggcgcatta

241 gttagttggt gaggtaacgg cccaccaaga cgatgatgcg tagccggcct gagagggtga
301 acggccacat tgggactgag acacggccca gactctacgg gaggcagcag tagggaattt
361 tcggcaatag gggcaaccct gaccgagcaa cgccgcgtga gtgaagacgg ccttcgggtt
421 gtaaaagctc ttgtttgtaa gggaagaacg gtagatagag aatatctaag tgacggtacc
481 ttaccagaaa gccacggcta actacgtgcc agcagccgcg gtaatacgta ggtggcgacc
541 gttatccgga attattggcc gtaaagggtg cgtaggcggc ctgttaagta agtggttaaa
601 ttgttgggct caacccaatc cagccactta aactggcagg ctagagtatt ggagaggcaa
661 gtggaattcc atgtgtagcg gtaaaatgcg tagatatatg gaggaacacc agtggcgaag
721 gcggcttgct agccaaagac tgacgctcat gcacgaaacc gtggggagcc aataggatta
781 gataccctag tagtccacgc cgtaaccgat gaatactaag tattggggaa actcagtgct
841 gcactaacgc aataagtatt ccgcctgtgg agtatgcacg caagtgtgaa acataaagga
901 attgacgggg gcccgcacaa gcggtggagt atgtggttta attcgacgca acgcgaagaa
961 ccttaccagg ccttgacatc ccttgcaaag ctgtagagat acagtagagg ttatcaagga
1021 gacaggtggt tgcatggtgt cgtcagctcg tgtcgtgaga tgttgggtta agtccggcaa
1081 cgagcgcaac ccttgccttc agttaccagc atttagttgg ggactctgga gggactgccg
1141 gtgataaacc ggaggaaggt ggggatgacg tcaaatcatc atgcccctta tggcctgggc
1201 tacacacgta ctacaatggc tgttacaacg tgcagcgacc tagcgatagg aagcgaatca
1261 ctaaaagaca gtctcagttc ggattgaagt ctgcaactcg acttcatgaa gctggaatcg
1321 ctagtaatcg cggatcagaa tgccgcggtg aatacgttct cgggccttgt acacaccgcc
1381 cgtcatacca tgagagctgg taatacccga ggccggtggc ctaaccgcaa ggaaggagcc
1441 gtcgaaggta ggactagtga ttggggtcaa gtcgtaacaa ggtaacc

> Actinomyces:

1 tcctgactca ggacgaacgc tgccggcgta cataacacat gcaagtcgaa cggtgaaggg
61 gcctgctttt gtgggtcctg gatgagtggc gaacgggtga gtaacacgtg agtaacctgc
121 ccccttcttc tggataaccg catgaaagtg tggctaatac gggatattct gggtctgtcg
181 catggtgggc ctgggaaaga ttgcgccttt tttggtgttt ttggtggggg atgggctcgc
241 ggcctatcag cttgttggtg gggtgatggc ctgccaaggc ggtgacgggt agccggcctg

301 agagggtgga cggtcacact gggactgaga cacggcccag actcctacgg gaggcagcag
361 tggggaatat tgcacaatgg gcgcaagcct gatgcagcga cgccgcgtga gggatggagg
421 ccttcgggtt gtgaacctct ttcgccagtg aagcaggcct gcctcgtttg tgggtgggtt
481 gacggtagct ggataagaag cgccggctaa ctacgtgcca gcagccgcgg taatacgtag
541 ggcgcgagcg ttgtccggaa ttattgggcg taaagagctc gtaggcggct ggtcgcgtct
601 gtcgtgaaat cctctggctt aactgggggc ttgcggtggg tacgggccgg cttgagtgcg
661 gtaggggaga ctggaactcc tggtgtagcg gtggaatgcg cagatatcag gaagaacacc
721 ggtggcgaag gcgggtctct gggccgttac tgacgctgag gagcgaaagc gtggggagcg
781 aacaggatta gataccctgg tagtccacgc cgtaaacgtt gggcactagg tgtgggggc
841 cttttccggg tcttccgcgc cgtagctaac gcattaagtg ccccgcctgg ggagtacggc
901 cgcaaggcta aaactcaaag gaattgacgg gggcccgcac aagcggcgga gcatgcggat
961 taattcgatg caacgcgaag aaccttacca aggcttgaca tgtgccggtc tgctccggag
1021 acggggtttc ctcctttgtg ggggctggtt cacaggtggt gcatggttgt cgtcagctcg
1081 tgtcgtgaga tgttgggtta agtcccgcaa cgagcgcaac ccttgtctcg tgttgccagc
1141 acgttgtggt ggggactcgc gggagactgc cggggtcaac tcggaggaag gtggggatga
1201 cgtcaaatca tcatgcccct tatgtcttgg gcttcacgca tgctacaatg gccggtacag
1261 agggctgcga taccgtgagg tggagcgaat cccttaaagc cggtctcagt tcggatcggt
1321 gtctgcaact cgacaccgtg aagttggagt cgctagtaat cgcagatcag caacgctgcg
1381 gtgaatacgt tctcgggcct tgtacacacc gcccgtcacg tcatgaaagt cggcaacacc
1441 cgaagcccgt

> Alistipes:

1 gatgaacgct agcggcaggc ttaacacatg caagtcgagg ggcagcacga ggtagcaata
61 ctttggtggc gaccggcgca cgggtgcgta acgcgtatgc aacctacctt taacaggggc
121 ataacactga gaaattggta ctaattcccc ataacattcg agaaggcatc ttcttgggtt
181 aaaaactccg gtggttaaag atgggcatgc gttgtattag ctagttggtg aggtaacggc
241 tcaccaaggc aacgatacat agggggactg agaggttaac ccccacatt ggtactgaga
301 cacggaccaa actcctacgg gaggcagcag tgaggaatat tggtcaatgg acgcaagtct

361 gaaccagcca tgccgcgtgc aggaagacgg ctctatgagt tgtaaactgc ttttgtacta
421 gggtaaacgc tcttacgtgt aggagcctga aagtatagta cgaataagga tcggctaact
481 ccgtgccagc agccgcggta atacggagga tccaagcgtt atccggattt attgggttta
541 aagggtgcgt aggcggtttg ataagttaga ggtgaaatac cggggctcaa ctccggaact
601 gcctctaata ctgttgaact agagagtagt tgcggtaggc ggaatgtatg gtgtagcggt
661 gaaatgctta gagatcatac agaacaccga ttgcgaaggc agcttaccaa actatatctg
721 acgttgaggc acgaaagcgt ggggagcaaa caggattaga taccctggta gtccacgcag
781 taaacgatga taactcgttg tcggcgatac acagtcggtg actaagcgaa agcgataagt
841 tatccacctg gggagtacgt tcgcaagaat gaaactcaaa ggaattgacg ggggcccgca
901 caagcggagg aacatgtggt ttaattcgat gatacgcgag gaaccttacc cgggcttgaa
961 agttagtgac gattctggaa acaggatttc ccttcggggc acgaaactag gtgctgcatg
1021 gttgtcgtca gctcgtgccg tgaggtgtcg ggttaagtcc cataacgagc gcaaccccta
1081 ccgttagttg ccatcaggtc aagctgggca ctctggcggg actgccggtg taagccgaga
1141 ggaaggtggg gatgacgtca aatcagcacg gcccttacgt ccggggctac acacgtgtta
1201 caatggtagg tacagagggc cgctaccccg cgaggggatg ccaatctcga aagcctatct
1261 cagttcggat cggaggctga aacccgcctc cgtgaagttg gattcgctag taatcgcgca
1321 tcagccatgg cgcggtgaat acgttcccgg gccttgtaca caccgcccgt caagccatgg
1381 aagctggggg tgcctgaagt tcgtgaccgc aaggagcgac ctagggcaaa accggtgact
1441 ggggct

The quantitative abundance is determined individually, from respective extracted DNA sequences, using a first set of probes and a second set of probes specific to each of the plurality of predetermined microbes associated with the saliva sample and the dental plaque sample respectively. The first set of probes includes a plurality of probes where each probe is utilized for each of the plurality of predetermined microbes (one probe for one predetermined microbe) associated with the saliva sample. Similarly, the second set of probes includes the plurality of probes where each probe is utilized for each of the plurality of predetermined microbes (one probe for one predetermined microbe) associated with the dental plaque sample.

In an embodiment, a multiplexed quantitative Polymerase Chain Reaction (qPCR) technique is employed for determining the quantitative abundance. More specifically, the multiplexed quantitative Polymerase Chain Reaction (qPCR) technique define a layout and arrangement of the plurality of probes that are of the first set of probes for determining the quantitative abundance associated with the saliva sample. Similarly, the multiplexed quantitative Polymerase Chain Reaction (qPCR) technique define the layout and arrangement of the plurality of probes that are of the second set of probes in the form of sequential runs for determining the quantitative abundance associated with the dental plaque sample.

More specifically, the first set of probes specific to each of the plurality of predetermined microbes associated with the saliva sample are utilized in two sequential multiplexed qPCR runs (defined by the multiplexed quantitative Polymerase Chain Reaction (qPCR) technique), to determine the quantitative abundance of each of the plurality of predetermined microbes associated with the saliva sample.

FIG. 3A illustrates an exemplary probe and multiplexed qPCR design for detecting and determining the quantitative abundance of each of the plurality of predetermined microbes associated with the saliva sample, according to some embodiments of the present disclosure. As shown in FIG. 3A, the two sequential multiplexed qPCR runs namely a first multiplexed qPCR run (Run 1) and a second multiplexed qPCR run (Run 2) are defined for determining quantitative abundance of each of the plurality of predetermined microbes associated with the saliva sample. Each Run of the first multiplexed qPCR run (Run 1) and the second multiplexed qPCR run (Run 2) includes five probes (hence it is also called as five-plex qPCR run) where each probe is utilized for the plurality of predetermined microbes associated with the saliva sample, from the list having: *Mogibacterium, Peptostreptococcus, Eubacterium, Solobacterium, Actinomyces,* and *Alistipe.* Also, each run of the first multiplexed qPCR run (Run 1) and the second multiplexed qPCR run (Run 2) contains a non-specific probe (denoted as 'Z' in FIG. 3A) at the start.

Further, as shown in FIG. 3A, the plurality of predetermined microbes, the quantitative abundance of which are being determined through the first multiplexed qPCR run are: *Mogibacterium, Peptostreptococcus, Eubacterium,* and *Solobacterium.* Further, the plurality of predetermined microbes, the quantitative abundance of which are being determined through the second multiplexed qPCR run are: *Mogibacterium, Peptostreptococcus, Actinomyces,* and *Alistipes.*

Similarly, the second set of probes specific to each of the plurality of predetermined microbes associated with the dental plaque sample are utilized in the two sequential multiplexed qPCR runs (defined by the multiplexed quantitative Polymerase Chain Reaction (qPCR) technique), to determine the quantitative abundance of each of the plurality of predetermined microbes associated with the dental plaque sample.

FIG. 3B illustrates an exemplary probe and multiplexed qPCR design for detecting and determining the quantitative abundance of each of the plurality of predetermined microbes associated with the dental plaque sample, according to some embodiments of the present disclosure. As shown in FIG. 3B, the two sequential multiplexed qPCR runs namely a third multiplexed qPCR run (Run 3) and a fourth multiplexed qPCR run (Run 4) are defined for determining quantitative abundance of each of the plurality of predetermined microbes associated with the dental plaque sample. Each Run of the third multiplexed qPCR run (Run 3) and the fourth multiplexed qPCR run (Run 4) includes five probes (hence it is also called as five-plex qPCR run) where each probe is utilized for the plurality of predetermined microbes associated with the dental plaque sample, from the list having: *Eubacterium, Dialister, Atopobium, Enterococcus, Mogibacterium,* and *Anaeroglobus.* Also, each run of the third multiplexed qPCR run (Run 3) and the fourth multiplexed qPCR run (Run 4) contains the non-specific probe (denoted as 'Z' in FIG. 3B) at the start.

Further, as shown in FIG. 3B, the plurality of predetermined microbes, the quantitative abundance of which are being determined through the third multiplexed qPCR run are: *Eubacterium, Dialister, Atopobium,* and *Enterococcus.* Further, the plurality of predetermined microbes, the quantitative abundance of which are being determined through the fourth multiplexed qPCR run are: *Eubacterium, Dialister, Mogibacterium,* and *Anaeroglobus.*

In an embodiment, the plurality of predetermined microbes associated with the saliva sample and the plurality of predetermined microbes associated with the dental plaque sample are captured from features of the respective pre-determined machine learning (ML) model. In an embodiment, the pre-determined machine learning (ML) model associated to the saliva sample is an ensemble machine learning (ML) model that is built using a microbial abundance data corresponding to a plurality of training saliva samples. The plurality of training saliva samples are the saliva samples used for training a machine learning model to obtain the corresponding pre-determined machine learning (ML) model. In an embodiment, the microbial abundance data corresponding to the plurality of training saliva samples is the quantitative abundance of all the microbes present in each of the plurality of training saliva samples.

Similarly, the pre-determined machine learning (ML) model associated to the dental plaque sample is the ensemble machine learning (ML) model that is built using the microbial abundance data corresponding to a plurality of training dental plaque samples. The plurality of training dental plaque samples are the dental plaque samples used for training the machine learning model to obtain the corresponding pre-determined machine learning (ML) model. In an embodiment, the microbial abundance data corresponding to the plurality of training dental plaque samples is the quantitative abundance of all the microbes present in each of the plurality of training dental plaque samples.

The ensemble ML model is built using the plurality of training biological samples consisting of saliva samples and dental plaque samples, individually, to obtain the corresponding pre-determined machine learning model. FIGS. 4A, 4B and 4C are flowcharts illustrating steps involved in building a pre-determined machine learning model according to some embodiments of the present disclosure. The technique for building the ensemble ML model accepts data in form of a feature table for multiple observations (the plurality of training biological samples) wherein each observation/ training biological sample is defined by 'N' features (F) which are either or both of continuous and counted variables with ($N \geq 1$). In case of training data (TR), each of the training biological samples/observations further have a preassigned class/category which is binary in nature, i.e., a healthy class (A) (e.g., affiliating to biological samples sourced from healthy (neurotypical) individuals i.e., with no symptoms/ history of ASD) and an unhealthy class or diseased class (B) (e.g., affiliating to biological samples sourced from individuals with symptoms of ASD or are diagnosed with ASD). In case of test data *(TS)* or data received during actual deployment of the method, the model(s) built based on training data predicts the class/category of the test biological samples/observations. During training process, the following steps are followed:

Initially at step 402, a healthy class tag or an unhealthy class tag is assigned to each of the training biological samples in the collected plurality of training biological samples (either saliva samples or dental plaque samples). The healthy class tag indicates samples sourced from healthy (neurotypical) individuals i.e., with no symptoms/ history of ASD, and the unhealthy class tag indicates samples sourced from individuals with symptoms of ASD or are diagnosed with ASD.

At step 404, the training data comprises of a plurality of microbial abundance profiles corresponding to each of the collected plurality of training biological samples, wherein each microbial abundance profile corresponding to a training biological sample comprises of one or a plurality of feature (s) and respective abundance value (s) of the feature (s), wherein each feature in the microbial abundance profile corresponds to one of a plurality of microbial taxonomic groups present in the plurality of training biological samples.

**In** the next step 406, the training data (TR) is randomly partitioned into two sets - namely, an internal-train *(ITR)* and an internal-test *(ITS),* based on a parameter '$L_1$', wherein $L_1$% training biological samples from the total training data constitute the *ITR* set and (100 - $L_1$) % of the training biological samples constitute the *ITS* set. Furthermore, the random partitioning into *ITR* and *ITS* sets is performed using a stratified sampling approach with the intent of preserving the relative proportion of training biological samples belonging to the healthy class (A) or the unhealthy class (B) in the total training data in these newly drawn subsets.

**In** the next step 408, a predefined number of subsets are randomly selected out of the internal training set based on a second parameter ($L_2$). Each of the subset comprises a randomly selected plurality of microbial abundance profiles corresponding to the plurality of training biological samples in the randomly selected subset, and wherein each of the subset comprises a proportionate part of training biological samples belonging to the healthy class (A) and the remaining training biological samples belonging to the unhealthy class (B). Thus, from *ITR, 'M'* randomly drawn subsets $ITRS_i$ (e.g., $ITRS_1$, $ITRS_2$, $ITRS_3$ .... $ITRS_M$), each containing S training biological samples are further generated, wherein S = $L_2$% of the training biological samples present in *ITR.* For example, the values of $L_2$ and M are 80 % and 100 respectively for present disclosure. Other values are within the scope of this invention.

**In** the next step 410, for each selected subset, a distribution of the abundance values of each of the features across the plurality of training biological samples in the selected subset, and the distribution of the abundance values of each of the features across the training biological samples belonging to the healthy class (A) in the selected subset and the training biological samples belonging to the unhealthy class (B) in the selected subset are noted. Thus, from each subset $ITRS_i$ (where $i$ = 1, 2, 3, ..., M), wherein there are total S training biological samples, each of which are described by *N* features ($F_j$) (where $j$ = 1, 2, 3, ..., *N),* the distributions of each of the features ($ITRS_iDF_j$) across S training biological samples are noted. Similarly, from each subset $ITRS_i$, wherein there are $S_A$ training biological samples belonging to the healthy class (A) and $S_B$ training biological samples belonging to the unhealthy class (B), each of the training biological samples being described by *N* features ($F_j$; $j$ = 1, 2, 3, ..., *N*), the distributions of each of the features ($ITRS_iD_AF_j$) across $S_A$ training biological samples, and the distributions of each of the features ($ITRS_iD_BF_j$) across $S_B$ training biological samples are noted.

In the next step 412, from the noted distributions of each selected subset, a first quartile value (Q1) and a third quartile value (Q3) of the distribution of each of the features is calculated across each of the plurality of training biological samples in the selected subset. In an example, the respective first quartile value (Q1) and the third quartile value (Q3) of $ITRS_iDF_j$ may also be referred as $Q1ITRS_iDF_j$ and $Q3ITRS_iDF_j$ .

Furthermore, in the next step 414, for each selected subset, a second quartile value of the distribution of each of the features across the training biological samples belonging to the healthy class ($Q2_A$) in the selected subset and the training biological samples belonging to the unhealthy class ($Q2_B$) in the selected subset is calculated. Thus, in an example, the median value (in other words, the second quartile value) of ($ITRS_iD_AF_j$) is referred as $Q2ITRS_iD_AF_j$, and the median value of ($ITRS_iD_BF_j$) is referred as $Q2ITRS_iD_BF_j$.

In the next step 416, for the M subsets of $ITRS_j$, a total of M values for each of $Q1ITRS_iDF_j$, $Q3ITRS_iDF_j$, $Q2ITRS_iD_AF_j$, and $Q2ITRS_iD_BF_j$, are calculated. Further at step 418, median value ( $\widetilde{Q1}_J$ ) is calculated for all calculated Q1, median value ( $\widetilde{Q3}_J$ ) is calculated for all calculated Q3, median value ( $\widetilde{Q2_{A_J}}$ ) is calculated for all calculated $Q2_A$ and median value ( $\widetilde{Q2_{B_J}}$ ) is calculated for all calculated $Q2_B$. Thus,

$$\widetilde{Q1}_J = \text{median of } \{Q1ITRS_1DF_j,\ Q1ITRS_2DF_j,\ Q1ITRS_3DF_j, \ldots\ Q1ITRS_MDF_j\}$$

$$\widetilde{Q3}_J = \text{median of } \{Q3ITRS_1DF_j,\ Q3ITRS_2DF_j,\ Q3ITRS_3DF_j, \ldots\ Q3ITRS_MDF_j\}$$

$$\widetilde{Q2_{A_J}} = \text{median } \{Q2ITRS_1D_AF_j, Q2ITRS_2D_AF_j, Q2ITRS_3D_AF_j, \ldots .\ Q2ITRS_MD_AF_j\}$$

$$\widetilde{Q2_{B_J}} = \text{median } \{Q2ITRS_1D_BF_j, Q2ITRS_2D_BF_j, Q2ITRS_3D_BF_j, \ldots .\ Q2ITRS_MD_BF_j\}$$

(where $i$ = 1,2,3, ... , *M*; *and* $j$ = 1, 2, 3, ... , *N*)

In the next step 420, a Mann-Whitney test is performed to test if a value of the feature ($F_j$) is significantly ($p<0.1$) different between the training biological samples belonging to the healthy class ($S_A$) and the training biological samples belonging to the unhealthy class ($S_B$) in each of the *M* randomly drawn subsets $ITRS_j$. Other statistical tests based on the nature of distribution (e.g., t-test for normal distribution), nature of sampling (e.g., Wilcoxon signed rank test for paired case and control samples) or other methods of statistical comparison relevant for microbiome datasets (e.g., ALDEx2) can also be adopted.

In the next step 422, the features are shortlisted based on a first predefined criteria utilizing calculated median values and the Mann-Whitney test. The first predefined criteria comprises if a feature $F_j$ is observed to have significantly (p<0.1) different values in $S_A$ compared to $S_B$ in more than 70% of $M$ subsets, and if $\widetilde{Q2_{A_J}} >= Q2_{min}$ OR $\widetilde{Q2_{B_J}} >= Q2_{min}$ (a predefined feature 'abundance' threshold and $Q2_{min}$ threshold as described in the case study). $F_j$ is added to a set of shortlisted features (*SF*).

In the next step 424, a set of features is generated using the shortlisted features (*SF*) using a second predefined criteria, wherein the set of features are less than or equal to 15. If the number of shortlisted features (*SF*) obtained in previous step satisfies the criteria $1 \leq SF \leq 15$, then the training process proceeds to model building with all the features in *SF*. If no shortlisted features *(SF)* are obtained in previous (i.e., $SF < 1$) then following step is performed with all the features $F_j$ for evaluating the ability of the features, when considered independently, to distinguish between training biological samples belonging to the healthy class (A) and the unhealthy class (B). Similarly, if the number of shortlisted features (*SF*) obtained in previous step exceeds fifteen ($SF > 15$) then following step is performed with all the shortlisted features *(SF)* for evaluating the ability of the features, when considered independently, to distinguish between the training biological samples belonging to the healthy class (A) and the unhealthy class (B).

Steps for shortlisting the features in case of $SF < 1$ or $SF > 15$: For each of the features (obtained previously) taken individually, different threshold values are used to classify the samples belonging to the set *ITR,* and the results are cumulated to construct a receiver operating characteristic curve (ROC curve) for each of the features. The area under the curve (AUC) of the ROC curve of any feature ($AUC^F$) is indicative of the utility of the feature to distinguish between the training biological samples belonging to the healthy class (A) and the unhealthy class (B), and the same is computed for every feature. The shortlisted features (*SF*) set is modified to include only the top fifteen features from a list of features arranged in a descending order of the $AUC^F$ values.

In the next step 426, a plurality of combinations of the features present in the set of features is created to generate corresponding plurality of candidate feature sets (CF), wherein the plurality of combinations of features comprises a minimum of one and a maximum of 15 features. In an embodiment, the maximum possible candidate feature sets that can be created in this process is $K = 2^{15} - 1 = 32767$ (i.e., maximum value of K = 32767).

In the next step 428, a plurality of candidate models is built corresponding to each of the plurality of candidate feature sets. At step 430, a model evaluation score (*MES*) is calculated corresponding to each of the plurality of candidate models. For each candidate feature set $CF_K$, a corresponding candidate model $CM_K$ is built and evaluated as mentioned in the steps mentioned below.

Steps for evaluating the candidate model:

• Step 1: The values of the features $F_j$ constituting a candidate feature set defining the training biological samples in *ITR* are transformed to $F_j'$ such that - $\widetilde{Q1}_J$, $\widetilde{Q3}_J$, $\widetilde{Q2_{A_J}}$ and $\widetilde{Q2_{B_J}}$

$$F_j' = 0 \quad \text{if } F_j < \widetilde{Q1}_J$$

$$F_j' = 1 \quad \text{if } F_j > \widetilde{Q3}_J$$

$$F_j' = 0.5 \quad \text{if } \widetilde{Q1}_J = \widetilde{Q3}_J$$

$$F_j' = \frac{F_j - \widetilde{Q1}_J}{\widetilde{Q3}_J - \widetilde{Q1}_J} \quad \text{if } \widetilde{Q1}_J < F_j < \widetilde{Q3}_J$$

• Step 2: If for a feature $F_j$, it is observed that $\widetilde{Q2_{B_J}} > \widetilde{Q2_{A_J}}$, then the feature $F_j$ is tagged as a 'numerator' feature and added to a set of numerator features $F_{numerator}$. Else, feature $F_j$ is tagged as a 'denominator' feature and added to a set of denominator features $F_{denominator}$.

• Step 3: Each candidate model ($CM_K$) is constituted as a simple ratio function given below -

$$CM_K = \frac{\sum F_{numerator}}{\sum F_{denominator}} \ldots \text{ when } F_{numerator} > 0 \text{ and } F_{denominator} > 0$$

or,

$$CM_K = \frac{\sum F_{numerator}+1}{\sum F_{denominator}+1} \ldots \text{ when either } F_{numerator} \text{ or } F_{denominator} = 0$$

wherein, $\Sigma F_{numerator}$ represents the sum of values of all numerator features for a particular sample, and, wherein, $\Sigma$ $F_{denominator}$ represents the sum of values of all denominator features for a particular sample.

For each of the features, a transformed value $F_j'$ as obtained above is used in the candidate model equation.

• Step 4: A candidate model c is used to generate candidate model scores (*$CMS_K$*) for each of the samples in the set *ITR*. From the set of scores *$CMS_K$*, the top 10 percentile and bottom 10 percentile scores are removed as outliers and thereafter the maximum and minimum scores from the set *$CMS_K$* are noted as *$CMS_{K_{max}}$* and *$CMS_{K_{min}}$* respectively.

• Step 5: Considering each of the scores in the set *$CMS_K$* as a threshold *(T)*, the model *$CM_K$* is used to (re)classify the samples in the training set *(ITR)* such that

◦ the training biological sample is classified into the healthy class (A) if *CMS >= T*

◦ or the training biological sample is classified into the unhealthy class (B) *if CMS < T*

and based on a comparison of these classifications and the true/original classes of the training biological samples, Matthew's correlation coefficients *(MCC)* for each of the thresholds are calculated, to evaluate how well each of the thresholds can distinguish between training biological samples between the healthy class (A) and the unhealthy class (B).

• Step 6: The threshold ($T_{max}$) which provides the maximum absolute *MCC* value ($|MCC_{max}|$) is noted. If $|MCC_{max}| < 0.4$ for a candidate model *$CM_K$*, then the candidate model is discarded from further evaluation. Else, the $|MCC_{max}|$ value is considered as the 'train- *MCC'* value ($|MCC_{train}|$) for the model *ITS* and the model and its corresponding $T_{max}$ threshold is used to classify the training biological samples in the internal-test set *(ITS)*. In another implementation of the process, the *$MCC_{max}$* threshold may not be applied for retaining the candidate model for subsequent evaluation. Before classifying the each of the training biological samples in the *ITS* set, the values of features characterizing the training biological samples of the *ITS* set are transformed using the method mentioned in step 418 while using the earlier obtained values of $\widetilde{Q1}_J$, $\widetilde{Q3}_J$, $\widetilde{Q2_{A_J}}$ and $\widetilde{Q2_{B_J}}$ from the *ITR* set.

• Step 7: The classification results on the training biological samples from the *ITS* set are compared against the true/original classes of the training biological samples (with pre-assigned labels), and the *MCC* for the model *$CM_K$* and its corresponding $T_{max}$ threshold on the *ITS* samples is calculated ($MCC_{test}$).

• Step 8: A model evaluation score *(MES)* for candidate model *$CM_K$* is calculated as $MES = |(MCC_{train} + MCC_{test})| - |(MCC_{train} - MCC_{test})|$

In the next step 432, the model *$CM_K$* is tagged as a "strong model" if all the features in the corresponding candidate feature set satisfies the Mann-Whitney test based shortlisting criteria described above. Otherwise, if any of the features in the corresponding feature set fails to satisfy the Mann-Whitney test, the model *$CM_K$* is tagged as a "weak model".

Further, the above process is repeated for candidate models and respective *MES* scores are used to rank all the models. The best model is subsequently chosen based on the *MES* score. In case there are more than one model with the best *MES* score, the best model is chosen based on the following criteria (in order of preference):

(a) the model with fewer number of features (i.e., based on a smaller candidate feature set) is chosen.

(b) the model with lower $T_{max}$ (threshold value) is chosen.

Further, the best model obtained through above steps is tagged as a forward model ($MD_{fwd}$). The model $MD_{fwd}$ additionally constitutes its corresponding $T_{max}$ threshold, the *$CMS_{K_{max}}$* and *$CMS_{K_{min}}$* values, and the $\widetilde{Q1}_J$, $\widetilde{Q3}_J$, $\widetilde{Q2_{A_J}}$ and $\widetilde{Q2_{B_J}}$ values corresponding to the *ITR* set.

In the next step 434, the tags assigned to the healthy class (A) and the unhealthy class (B) of the plurality of samples present in the training data are swapped. At step 436, all of the above steps 404 to 432 to determine the best model are repeated after swapping the class labels (A <-> B) for the entire training set *(TR)* to obtain a best model tagged as the reverse model ($MD_{rev}$). The model ($MD_{rev}$) additionally constitutes its corresponding $T_{max}$ threshold, the *$CMS_{K_{max}}$* and *$CMS_{K_{min}}$* values, and the $\widetilde{Q1}_J$, $\widetilde{Q3}_J$, $\widetilde{Q2_{A_J}}$ and $\widetilde{Q2_{B_J}}$ values corresponding to the *ITR* set.

At step 438, a plurality of forward models and a plurality of reverse models are generated by repeating step (404) through (436) for a predefined number of times using randomly partitioned internal training set and the internal test set. The steps (404) through (436) are iterated 'R' times using multiple randomly partitioned *ITR* and *ITS* sets generated initially. After each iteration, (i) the features constituting the models $MD_{fwd}$ and the models $MD_{rev}$ obtained in the current iteration *(r)* are compared against, and if necessary, appended to, a set of unique features $F_{unq}$ that consists of respective features constituting the $MD_{fwd}$ and $MD_{rev}$ obtained in earlier iterations (i.e., up to iteration r - 1). After 'R' iterations, a plurality of forward models and a plurality of reverse models are generated for a predefined number of times using randomly partitioned internal training set and the internal test set. The iterations proceed while the value of R satisfies the following criteria -

(i)

$$R \leq R_{max}$$

(ii)

$$(|F_{unq}| \text{ after iteration R}) > (|F_{unq}| \text{ after iteration } R - R_{unq})$$

(iii)

$$|F_{unq}| \text{ after iteration no. } R \;<=\; Fet_{max}$$

Wherein, $R_{max}$ is a parameter indicating the maximum number of iterations allowed;
$R_{unq}$ is a parameter indicating the maximum number of iterations allowed without any cumulative increase in the number of unique features $|F_{unq}|$ in the models being generated in consecutive iterations; and
$Fet_{max}$ is a parameter indicating the maximum allowed value of $|F_{unq}|$ (i.e., the no. of unique features cumulated through the iterative process).

**In** an embodiment, the exemplary values of $R_{max}$, $R_{unq}$, and $Fet_{max}$ are 100, 10, 100 respectively for the present disclosure. Other values of these and other parameters here for finetuning and suitability for other datasets are within the scope of the present invention.

**In** the next step at 440, an ensemble of forward models is generated using the plurality of forward models and an ensemble of reverse models is generated using the plurality of reverse models. This is referred as an ensemble of forward models (ENS-$MD_{fwd}$)) and an ensemble of reverse models (ENS-$MD_{rev}$).

At step 442, the best models from each of these ensembles, i.e., the best of the forward models ($BMD_{fwd}$) and the best of the reverse models ($BMD_{rev}$) respectively, are identified.

If all models in an ensemble are weak models, the best model from the ensemble *(BMD)* is chosen by ranking the models based on their model evaluation scores and associated criteria. Also, if an ensemble contains more than one strong model, then only those strong models are considered for ranking based on their model evaluation scores and associated criteria as mentioned above, and the best model from the ensemble (*BMD*) is thereby chosen.

**In** the next step 444, a final single model ($FM_{single}$) is chosen as the ensemble classification model from amongst the best forward model and the best reverse model based on how they classify the individual samples from the training data. Once the best models from each of the ensemble of forward models and the ensemble of reverse models, i.e., the best of the forward models ($BMD_{fwd}$) and the best of the reverse models ($BMD_{rev}$) are identified, the final single model ($FM_{single}$) is chosen from amongst $BMD_{fwd}$ and $BMD_{rev}$ based on how well they can classify the individual training biological samples from the entire training set (TR). The AUC value for ROC curves for each of these two models are computed based on the predicted model scores for the training set (TR) samples and their pre-assigned classes (the healthy class (A) and the unhealthy class (B)). The model having the best AUC for ROC value is selected as the final single model ($FM_{single}$). If both $BMD_{fwd}$ and $BMD_{rev}$ have the same AUC value, $BMD_{fwd}$ is chosen as $FM_{single}$.

**In** an alternate implementation $FM_{single}$ can be chosen based whether $BMD_{fwd}$ or $BMD_{rev}$ obtains a higher *MCC* value while classifying the TR training biological samples. Once the $FM_{single}$ model has been chosen, for classification of any samples from a test set *(TS)* or any sample data received during actual deployment, the $FM_{single}$ model is used after:

(a) appropriately transforming the features corresponding to the training biological sample being classified using the $\widetilde{Q1}_J$, $\widetilde{Q3}_J$, $\widetilde{Q2_{A_J}}$ and $\widetilde{Q2_{B_J}}$ values corresponding to the $FM_{single}$ model,

(b) limiting the model score between a maximum of $CMS_{K_{max}}$ and a minimum of $CMS_{K_{min}}$ values corresponding to the

$FM_{single}$ model, and
(c) classification based on the model score using its corresponding threshold $T_{max}$.

According to an embodiment of the disclosure, the ensemble of forward models (ENS-$MD_{fwd}$) and the ensemble of reverse models (ENS-$MD_{rev}$) are also evaluated for their collective classification efficiencies using an ensemble model scoring. **In** the ensemble scoring method, each of the models *(MD)* constituting an ensemble (ENS) are used to generate a model score *(MS)* for each of the samples from the entire TR set. For any specific training biological sample, the values of the features corresponding to the training biological sample are appropriately transformed using the $\widetilde{Q1}_J$, $\widetilde{Q3}_J$, $\widetilde{Q2_{A_J}}$ and $\widetilde{Q2_{B_J}}$ values corresponding to the model MD. The model scores *(MS)* are then transformed into scaled model scores *(SMS)* having values between **-1** and +1, using the following procedure:

$$SMS = (MS - T_{max})/(\ CMS_{K_{max}} - T_{max}), \ \ldots.. \text{ when } MS >= T_{max},$$

and

$$SMS = (MS - T_{max})/(\ T_{max} - CMS_{K_{min}}), \ \ldots.. \text{ when } MS < T_{max},$$

Wherein, $T_{max}$, $CMS_{K_{max}}$, and $CMS_{K_{min}}$ values corresponding to the respective model is used.

Let $SMS_{avg}$ be the average of all *SMS* obtained using all models in ENS for a particular sample.

When using Forward model [ENS-$MD_{fwd}$],

$$SMS_{avg} = SMS_{avg} * (+1)$$

If $SMS_{avg}$ >= 0, sample is classified as the unhealthy class (B)
If $SMS_{avg}$ < 0, sample is classified as the healthy class (A)
When using Reverse model [ENS-$MD_{rev}$]:

$$SMS_{avg} = SMS_{avg} * (-1)$$

If $SMS_{avg}$ > 0, sample is classified as the unhealthy class (B)
If $SMS_{avg}$ <= 0, sample is classified as the healthy class (A)

If all models in one of the ensembles are weak models, then the other one having (one or more) strong models is selected as a final ensemble model ($FM_{ens}$), and subsequently used for classification of any of training biological samples from a test set *(TS)* or any sample data received during actual deployment of the method, using the scoring and classification process mentioned in above paragraph. If both ensembles have constituent strong models, then both the ensembles are evaluated for their efficiency by scoring them on all individual samples in *TR.* The AUC value for ROC curves for each of these two ensembles are computed based on the predicted $SMS_{avg}$ for all the training set (TR) samples and their pre-assigned classes. The ensemble of models having the best AUC for ROC value is selected as the final ensemble model ($FM_{ens}$). In case both ENS-$MD_{fwd}$ and ENS-$MD_{rev}$ exhibit equal AUC values then ENS-$MD_{fwd}$ is chosen as the final ensemble model ($FM_{ens}$). In an alternate implementation, $FM_{ens}$ can be chosen based whether ENS-$MD_{fwd}$ and ENS-$MD_{rev}$ obtains a higher average MCC value for their respective constituent models while classifying the TR samples.

Thus, either the $FM_{single}$ model or $FM_{ens}$ ensemble of models can be used for classification of any of training biological samples from a test set (*TS*) or any training biological sample data received during actual deployment.

In an embodiment, one or more predetermined microbes out of the plurality of predetermined microbes associated with the saliva sample, are common to the first multiplexed qPCR run (Run 1) and the second multiplexed qPCR run (Run 2) for determining the associated quantitative abundance. In an embodiment, the one or more predetermined microbes that are common to the first multiplexed qPCR run (Run 1) and the second multiplexed qPCR run (Run 2) are determined based on (i) a median abundance (obtained from microbial abundance data) of each of the plurality of predetermined microbes obtained from the plurality of training saliva samples, (ii) a frequency of occurrence of each of the plurality of predetermined microbes constituting the ensemble ML model associated with the saliva sample. More specifically, the one or more predetermined microbes (from amongst the set of predetermined microbes) has/ have the highest (or relatively higher) median abundance or frequency of occurrence (as compared to the median abundance(s) or the frequency of occurrence of each microbe in the remaining set of predetermined microbes) across the plurality of training saliva samples is/ are common to the first multiplexed qPCR run (Run 1) and the second multiplexed qPCR run (Run 2).

For example, a predetermined microbe having a high median abundance or a high frequency of occurrence from the microbial abundance data is determined and utilized in more than one Run. As shown in FIG. 3A, the predetermined microbe *Mogibacterium* is common for both the first multiplexed qPCR run (Run 1) and the second multiplexed qPCR run (Run 2). Similarly, the predetermined microbe *Peptostreptococcus* is also common for both the the first multiplexed qPCR run (Run 1) and the second multiplexed qPCR run (Run 2).

Similarly, the one or more predetermined microbes out of the plurality of predetermined microbes associated with the dental sample, are common to the third multiplexed qPCR run (Run 3) and the fourth multiplexed qPCR run (Run 4) for determining the associated quantitative abundance. In an embodiment, the one or more predetermined microbes that are common to the third multiplexed qPCR run (Run 3) and the fourth multiplexed qPCR run (Run 4) are determined based on (i) the median abundance (obtained from microbial abundance data) of each of the plurality of predetermined microbes obtained from the plurality of training dental plaque samples, (ii) the frequency of occurrence of each of the plurality of predetermined microbes constituting the ensemble ML model associated with the dental plaque sample. More specifically, the one or more predetermined microbes (from amongst the set of predetermined microbes) has/ have the highest (or relatively higher) median abundance or frequency of occurrence (as compared to the median abundance(s) or the frequency of occurrence of each microbe in the remaining set of predetermined microbes) across the plurality of training dental plaque samples is/ are common to the third multiplexed qPCR run (Run 3) and the fourth multiplexed qPCR run (Run 4).

For example, a predetermined microbe having a high median abundance or a high frequency of occurrence from the microbial abundance data is determined and utilized in more than one Run. As shown in FIG. 3B, the predetermined microbe *Eubacterium* is common for both the third multiplexed qPCR run (Run 3) and the fourth multiplexed qPCR run (Run 4). Similarly, the predetermined microbe *Dialister* is also common for both the third multiplexed qPCR run (Run 3) and the fourth multiplexed qPCR run (Run 4).

In an embodiment, the quantitative abundance determination involves creating abundance or feature table and generation of the percent normalized abundance or feature table having percent normalized abundance values of the predetermined microbes or OTUs or taxas in each sample. In another embodiment, Multicolour Combinatorial Probe Coding (MCPC) qPCR or real-time PCR based measurement of abundance of the microbial OTUs or taxas can also be considered for quantification of a predefined set of taxas. Alternatively, any other pre-processing techiniques or data normalization techniques known in the state of art can be used for normalization and feature selection from the main feature table.

**Design configuration & number of** multiplexed **qPCR runs required for quantifying the abundance of target microbes or microbial taxonomic groups or microbial taxa/ features:**

The quantitative abundance of each of the microbial taxonomic groups or microbes, that are common to each of the multiplexed qPCR runs (the first multiplexed qPCR run, and the second multiplexed qPCR run), is determined based on a normalizing factor *($NF_{run}$)* associated with each multiplexed qPCR run and the quantitative abundance of associated microbial taxonomic group in the corresponding multiplexed qPCR run.

For example, considering a maximum of five unique DNA fragments, each representing a microbial taxa or spike DNA, can be quantified in a one multiplexed qPCR run. Therefore, to analyze a disease signature (captured in an ML model) comprising of 'n' microbial taxa/ features, a minimum of (1 + [(n - 4)/4]) multiplexed qPCR runs would be required wherein *'n'* is the unique number of microbial taxonomic groups constituting the frugal set of markers, and wherein each multiplexed qPCR run is configured to determine, in the test biological sample, the relative abundance of a predetermined subset of the microbial taxonomic groups constituting the disease signature. This minimum number is based on assumptions that:

(a) the spike DNA should be analyzed at least once in one of the '(1 + [(n - 4)/41)' multiplexed qPCR runs; and
(b) an overlap of at least one microbial taxa/ features was done between two corresponding runs.

For example, if a disease signature comprises of 8 microbial taxa (A, B, C, D, E, F, G, and H), then at least TWO multiplexed qPCR runs would be required, where Z is the spike DNA of known concentration and taxa 'D' is analyzed in both multiplexed qPCR runs. Here, $\lceil (n-4)/4 \rceil$ indicates a ceiling value of the expression. Thus, the minimum no. of required qPCR runs would be:

1 for 1-4 signatures/ features
2 for 5-8 signatures/ features
3 for 9-12 signatures/ features
4 for 13-16 signatures/ features, and so on...

**Example A: Run 1:** Z A B C **D; Run2: D** E F G H

Similarly, for a feature size of 12 (A, B, C, D, E, F, G, H, I, J, K, and L), at least THREE multiplexed qPCR runs would be required, where Z is the spike DNA of known concentration and taxa 'D' and 'H' are analyzed in twice.

**Example B: Run 1**: Z A B C **D; Run 2**: **D** E F G **H; Run 3: H** I J K L

If the number of features constituting the signature is not optimal for the above condition, i.e., for e.g., the number of features is 10, then more than one microbial taxon can be analyzed twice. The same is exemplified below, wherein taxa C and D are analyzed twice (in Runs 1 and 2). Similarly, taxa F and G are also analyzed twice (in Runs 2 and 3).

**Example C: Run 1:** Z A B **C D; Run 2: C D** E **F G; Run3: F G** H I J

In alternate implementations, the spike DNA (Z) can be analyzed in each of the runs. In that scenario, the first multiplexed qPCR will be able to accommodate up to FOUR features. Each additional multiplexed qPCR run will accommodate up to THREE new/ additional features as shown by underlining in the example below. Thus, two multiplexed qPCR runs would be required for a feature set of up to seven; three qPCR runs for a feature set of up to ten and so on.

**Run 1: Z** A B C **D** ; **Run 2: Z D** E F **G** ; **Run 3: Z G** H I J

Furthermore, if the number of features is not optimal for the above condition, then two or more taxa/ features can be analyzed multiple times as shown in example C.

**Methodology to interpret/ quantify the abundance of a microbial taxon or microbes or microbial taxonomic groups from data obtained from above qPCR configurations:**

Given that the concentration of the spike DNA (*Z*) is previously known - say $X_1$. If the measured concentration of *Z* in the multiplexed qPCR is $X_2$, then all the measured concentration in a single multiplexed qPCR run can be normalized multiplying by a normalizing factor ($NF_{run}$) of $X_1/X_2$.

In cases where the spike DNA is only analyzed in only one of the multiplexed qPCR runs (as shown in examples A, B and C), then the normalized values of the taxa/ feature in the first run which is/ are re-analyzed in the Run 2, can be used for adjusting the concentrations inferred from the Run 2 of the multiplexed qPCR. Following Example-A (described previously),

Actual conc of *Z*: $X_1$
Measured conc of *Z*: $X_2$
Normalizing factor $NF_{run1}$: $X_1/X_2$
Inferred conc. of A (from Run 1): $A'_{run1} \times NF_{run1}$
Inferred conc. of B (from Run 1): $B'_{run1} \times NF_{run1}$
Inferred conc. of C (from Run 1): $C'_{run1} \times NF_{run1}$
Inferred conc. of D (from Run 1): $D'_{run1} \times NF_{run1}$

Where $A'_{run1}$, $B'_{run1}$, $C'_{run1}$, and $D'_{run1}$ are the measured/ analyzed concentrations of taxa/ feature A, B, C and D respectively.
Normalizing factor $NF_{run2}$: Inferred conc. of D from Run 1/ Measured concentrations of feature D in Run 2

Inferred conc. of E: $E'_{run1} \times NF_{run2}$
Inferred conc. of F: $F'_{run1} \times NF_{run2}$
Inferred conc. of G: $G'_{run1} \times NF_{run2}$
Inferred conc. of H: $H'_{run1} \times NF_{run2}$

The same protocol may be repeated for normalizing/ adjusting the concentrations measured from all subsequent runs (as in example B). In case wherein more than once feature is analyzed in subsequent runs (as in example C), a median Normalizing factor *(NF)* - derived from the *NFs* for each of the replication features may be used for computing the inferred concentrations from that run.

In alternate implementations, wherein the spike DNA (Z) is analyzed in each of the runs (as in example D), Normalizing factor *(NF)* corresponding to each of the runs may be computed and used for inferring the concentrations of the constituent features. In cases, where the measured spike DNA (Z) concentration varies by more than 25% from the actual concentration, it is suggested that the observations from the said multiplexed qPCR run be discarded, and a fresh multiplexed qPCR run for the sub-set of features be performed.

In an alternate implementation using multiplexed qPCR runs, the marker feature (marker microbe or taxa) having the lowest variance in relative abundance in training data across both the classes, is selected as the anchor marker (*AM*), and the relative abundance of each of the markers is computed by multiplying the ratio of their estimated/inferred DNA concentrations and the estimated/inferred DNA concentration of *AM* with the median abundance of *AM* across all training data. For example, if the marker features are A, B, C and D. wherein A is the anchor marker (*AM*) having a median abundance of $ABN_{AM}$, then the abundances of the marker features B, C and D will be computed as;

$$ABN_B = (Inferred\ conc.\ of B / Inferred\ conc.\ of A) \times ABN_{AM}$$

$$ABN_C = (Inferred\ conc.\ of C / Inferred\ conc.\ of A) \times ABN_{AM}$$

$$ABN_D = (Inferred\ conc.\ of D / Inferred\ conc.\ of A) \times ABN_{AM}$$

At step 208 of the method 200, the quantitative abundance is collated through the abundance determining module 112. The quantitative abundance of (i) each of the plurality of predetermined microbes associated with the saliva sample and (ii) each of the plurality of predetermined microbes associated with the dental plaque sample, determined at step 206 of the method 200 is collated to obtain a hybrid abundance matrix.

At step 210 of the method 200, a model score is determined based on the hybrid abundance matrix obtained at step 208 of the method 200, through the ML module 114. The ML module 114 includes a pre-determined machine learning (ML) model explained and obtained at step 206 of the method 200 is employed to determine the model score based on the hybrid abundance matrix.

At step 212 of the method 200, the risk assessment of the subject is performed through the assessment module 116. The risk assessment is performed based on the model score obtained at step 210 of the method 200 and a predefined threshold value. For example, if the model score obtained at step 210 of the method 200 is greater than or equal to the predefined threshold value, then the subject is assessed to be having the autism spectrum disorder. If the model score obtained at step 210 of the method 200 is less than the predefined threshold value, then the subject is assessed as not having the autism spectrum disorder.

At step 214 of the method 200, a personalized recommendation for the subject assessed as having autism spectrum disorder at step 212 of the method 200 is designed through the recommendation module 118. In an embodiment, the personalized recommendation includes utilizing a set of rules for the set of microbes that constitute the pre-determined machine learning model to identify one or more personalized probiotic and antibiotic candidates that may be employed to ameliorate disease symptoms in the subject identified as having autism spectrum disorder. In an embodiment, the microbes (organisms) contributing to generation of model score at step 210 are mapped to a predefined set of antibiotic and probiotic candidates and appropriate personalized targets for treatment and recommendation are identified accordingly.

More specifically, one or a combination of probiotic and antibiotic (microbial) candidates or targets designed via or based on features (i.e., taxa or microbes) constituting the ML model. The designing of the one or the combination of probiotic and antibiotic candidates' targets may be performed by mapping the features (i.e., organisms/taxa/ microbes) constituting the ML model to the complete set of microbes (or a pre-defined subset of the same) using the following steps

At step 1, pair-wise correlations (using the Pearson's and/or spearman's correlation index) are computed between abundances of features (i.e., organisms/ taxa/ microbes) constituting the ML model and the abundances corresponding to the complete set of microbes computed individually from (a) the subset of biological samples corresponding to the healthy class i.e. the class of samples that are taken from individuals not having ASD, and (b) the diseased class i.e. the class of samples that are taken from individuals having ASD). Wherein the samples belonging to the healthy and diseased classes are used as training data for generating the ML model.

At step 2, positive and negative interactions between features (i.e., organisms/ taxa/ microbes) constituting the ML model and all other taxa in the healthy and the diseased class of training samples (individually) are deduced using critical correlation (r) value as the cut-off (as taught in Batushansky et al., 2016), such that inter-taxa correlation index values greater than +r value are affiliated as 'positive interactions', while those less than *-r* value are affiliated as 'negative interactions'.

At step 3, the steps 1 and 2 are repeated 1000 times and only those interactions are considered relevant that appear in at least 70% of iterations with a BH (Benjamini-Hochberg) corrected p-value cut-off of 0.1 are retained (hereafter referred to as model taxa interactions corresponding to health and diseased class of samples).

At step 4, thereafter, following set of rules (indicated in Table 1 below) are used to arrive at the relevant candidate using the retained model taxa interactions:

Table 1

| Probiotic Candidates | Antibiotic Candidates |
|---|---|
| $(M_H - C_T)_{HP}$ && $(M_H - C_T)_{DP}$<br>$(M_H - C_T)_{DP}$<br>$(M_D - C_T)_{HN}$ && $(M_D - C_T)_{DN}$ | $(M_H - C_A)_{HN} \| (M_H - C_A)_{DN}$<br>$(M_D - C_A)_{HP} \| (M_D - C_A)_{DP}$ |

From Table 1,

$M_H$ represents a model taxon having significantly higher abundance in healthy class;
$M_D$ represents a model taxon having significantly higher abundance in diseased (unhealthy) class;
$C_T$ represents a potential candidate for recommendation;
$C_A$ represents a potential antibiotic target candidate;
$M_H$ - $C_T$ represents an interaction between a model taxon (abundant in healthy class) with a potential candidate for recommendation;
$M_D$ - $C_T$ represents an interaction between a model taxon (abundant in diseased class) with a potential candidate for recommendation;
$M_D$ - $C_A$ represents an interaction between a model taxon (abundant in diseased class) with a potential antibiotic target candidate;
$M_H$ - $C_A$ represents an interaction between a model taxon (abundant in healthy class) with a potential antibiotic target candidate;
*HP* represents a positive interaction in a healthy environment population;
*HN* represents a negative interaction in a healthy environment population;
*DP* represents a positive interaction in a diseased environment population; and
*DN* represents a negative interaction in a diseased environment population.

One or more of the set of microbes constituting the identified probiotic candidates may be recommended (individually or in combination) as probiotic formulations for treating (or ameliorating the symptoms of or the disease severity of) individuals identified as having ASD. Furthermore, the mentioned probiotic formulations may help in promoting development of a healthy oral microbiome (in the individuals administered with the probiotic) which may be employed to ameliorate the symptoms of, or the disease severity of individuals identified as having ASD.

One or more of the set of microbes constituting the identified antibiotic microbial candidates may be targeted (individually or in combination) via antibiotics or other treatment methodologies that can reduce the abundance of the identified antibiotic microbial candidate(s) and may be recommended for ameliorating the symptoms of or the disease severity of individuals identified as having ASD. Furthermore, such antibiotic recommendation (as detailed above) may also help in promoting development of a healthy oral microbiome, which (may) ameliorate the symptoms of, or the disease severity of individuals identified as having ASD.

Further, a kit for risk assessment of autism spectrum disorder in the subject, is disclosed. FIG. 5 illustrates an exemplary block diagram of a kit 500 for risk assessment of autism spectrum disorder present in the subject, according to some embodiments of the present disclosure. As shown in FIG. 5, the kit 500 includes an input module 502, one or more hardware processors 504 and an output module 506. The input module 502 is used for receiving the saliva sample and the dental plaque sample of the subject whose risk of autism spectrum disorder is to be assessed. In an embodiment, the input module 502 may be a medium, a carrier, a set of mediums, or a set of carries that can hold the saliva sample and the dental plaque sample individually.

The one or more hardware processors 506 are configured to analyze the saliva sample and the dental plaque sample present in the input module 502, using the one or more steps of the method. In an embodiment, the one or more hardware processors 506 are equivalent or same that of the one or more hardware processors 106 of the system 100. The output module 506 is used for displaying the risk assessment of autism spectrum disorder of the subject, based on the analysis of the one or more hardware processors 506. In other words, the output module 506 is used for indicating on the presence or non-presence of the ASD of the subject. In an embodiment, the output module 506 includes but are not limited to a display device, an indicator, a color indicator, or any other equipment that can show the result representation on the ASD to the subject.

The embodiments of the present disclosure provides a mechanism for identifying the risk assessment of autism spectrum disorder of the subject by making use of paired oral microbial samples of both the saliva sample and the dental plaque sample. The present disclosure determines minimum number of microbes, or OTUs or taxonomies for determining the microbial quantitative abundance using which the risk assessment of the subject for the ASD is identified. More specifically, only the five microbes, or OTUs or taxonomies *(Mogibacterium, Peptostreptococcus, Eubacterium, Solobacterium, Actinomyces,* and *Alistipes)* of the saliva sample, and the only the five microbes, or OTUs or taxonomies *(Eubacterium, Dialister, Atopobium, Enterococcus, Mogibacterium,* and *Anaeroglobus)* of the saliva sample, and in total only the 10 OTUs or taxonomies that are more influenced are identified for determining the microbial quantitative abundance. Hence, the present disclosure requires less resources, simple and yet effective.

The present disclosure is not based on psychiatric analysis and behavioral checklists. It is rather a systematically designed, exhaustively tested and repeatedly validated quantitative methodology for ASD risk assessment and guided therapeutic development.

The present disclosure focuses on oral microbiome of ASD subjects, an avenue which is sparsely touched upon. Dependence on a large number of microbial features for developing the assessment procedure is neither easily translatable nor economical. Moreover, it is difficult to arrive at a relevant and relatable therapeutic solution based on such a large number of microbes in a diagnostic marker. The present disclosure relies on creation of co-related hybrid features (OTU) for each subject through paired sampling. And the disclosed biomarker model is constituted by as less as 10 OTUs taken together from both the saliva sample (5 OTUs) and the dental plaque ample (5 OTUs) microbiome samples with an average AUC of 0. 88$\pm$0.10 with models giving a held-out AUC of as high as 0.98 for ensemble model developed using hybrid feature set. In hybrid feature based models, the OTUs are not independently considered (unlike the competitive literature) but combined together to create hybrid biomarkers constituted by site-specific microbes. Even site-specific biomarkers are consistently efficient as well as sparse/frugal through the present disclosure. The data splits employed are different which are expected to yield differences in performance, however the smaller number of features with good average cross validated performance is a clear differentiator.

By focusing on frugal combination of disease biomarkers, the present disclosure enables accurate and easy diagnosis of a disease. Importantly, selection of a small set of OTUs (total 10 OTUs taken together from both the saliva sample and the dental plaque sites in a single hybrid ensemble model) as biomarker component. The present disclosure further enables an easy trace-back towards potential causality and guides in focused personalized recommendation design. A systematic diagnostic and personalized recommendation design, with a small set of features, makes it convenient and economical to deploy for mass adoption.

The disclosure provides biomarkers using the described methods employed on the public dataset provided in NCBI SRA database study ID SRP097646. The described methodology can be adopted for other datasets to arrive at additional region/age or other metadata specific models and recommendations.

Example scenario:

A. Model training: The ML model along with the set of ensemble models are obtained using the data associated with the respective samples. i.e., saliva samples, or dental plaque samples, or both. The data associated with the respective samples is divided into training data and the test data. The present disclosure accepts data in form of a feature table for multiple observations (or samples) wherein each observation/ sample is defined by '*N*' features (*F*) which are continuous variables and *(*$N \geq 1$). **In** case of training data (*TR*), each of the samples/observations further have a preassigned class/category which is binary in nature (e.g., A or B). **In** case of test data *(TS)* or data received during actual deployment of the method, the model(s) built based on training data predicts the class/category of the samples/observations.

B. Model training results: The features (microbes) of the single best model and the ensemble model are analyzed and the features that are most frequently occurred are identified, which are then used as the plurality of predetermined microbes to determine the quantitative abundance in real test cases, as explained at step 206 of the method 200.

Table 2 shows the list of features (microbes) of the ensemble model for the hybrid samples (both saliva and dental plaque samples) along with their occurrences. In table 2, SL mean the microbe is associated saliva sample and PQ mean the microbe is associated with the dental plaque sample.

Table 2

| Unique Features in Ensemble Model | Occurrence |
|---|---|
| SL_Mogibacterium | 10 |
| PQ_Eubacterium | 9 |
| PQ_Dialister | 7 |
| PQ_Atopobium | 3 |
| PQ_Enterococcus | 2 |
| SL_Peptostreptococcus | 2 |
| PQ_Mogibacterium | 2 |
| SL_Eubacterium | 2 |
| SL_Solobacterium | 1 |
| SL_Actinomyces | 1 |
| PQ_Anaeroglobus | 1 |
| SL_Alistipes | 1 |

Table 3 shows the list of features (microbes) of the ensemble model for the saliva sample along with their occurrences.

Table 3

| Unique Features in Ensemble Model | Occurrence |
|---|---|
| Alloprevotella | 10 |
| Parvimonas | 7 |
| Peptostreptococcus | 6 |
| Anaeroglobus | 4 |
| Morococcus | 4 |
| Solobacterium | 3 |
| Peptococcus | 2 |
| Allisonella | 1 |
| Mesocricetibacter | 1 |
| Anaerovorax | 1 |

Table 4 shows the list of features (microbes) of the ensemble model for the dental plaque sample along with their occurrences.

Table 4

| Unique Features in Ensemble Model | Occurrence |
|---|---|
| Mogibacterium | 10 |
| Mesocricetibacter | 10 |
| Catonella | 5 |
| Peptostreptococcus | 5 |
| Anaerovorax | 5 |
| Solobacterium | 4 |
| Parvimonas | 2 |

Table 5 shows an exemplary model metrics data of the single best ML model and the ensemble model that has performed the best for the hybrid model using both saliva sample and dental plaque sample.

Table 5

| Model Metrics | Single best ML Model | Ensemble ML model |
|---|---|---|
| CV (100 iterations) Mean AUC | 0.85 | 0.8792 |
| AUC Min-Max | 0.60-1.00 | 0.64-1.00 |
| AUC Std. Dev | 0.110104 | 0.095976 |
| CV (100 iterations) Mean MCC | 0.516945 | 0.580859 |
| MCC Min-Max | 0.00-1.00 | 0.00-1.00 |
| MCC Std. Dev | 0.258161 | 0.24416 |
| Training AUC | 0.95619 | 0.958095 |
| Training MCC | 0.741941 | 0.826645 |

C. Case Study: A case study is conducted on a subject for whom the risk of ASD to be ascertained and the steps 1 to 8 are mentioned to explain the case study and the steps are in line with the steps of the method 200 of the present disclosure.

At step 1, both the saliva sample and dental plaque samples are collected at the same, as test samples from the subject for whom the risk of ASD to be ascertained.

At step 2, the raw abundances of various microbial taxonomic groups present in the collected samples are quantified and a unified table is created. Methodology used in this step involves extraction of microbial DNA contents from the collected samples followed by amplification and sequencing of either full-length or specific variable regions of the bacterial 16S rRNA marker genes using the next-generation sequencing platform or by using the multiplexed qPCR-based quantification methodology. Table 6 shows the raw abundance of various microbial taxonomic groups present in the collected samples. In Table 6, PQ refers to microbial taxonomic groups related to dental plaque sample and SL refers to microbial taxonomic groups related to saliva sample.

Table 6

| Microbial taxonomic groups | Raw abundance |
|---|---|
| PQ_Neisseria | 112 |
| PQ_Morococcus | 23 |
| PQ_Kingella | 46 |
| PQ_Eikenella | 5 |
| ... | .... |
| ... | .... |
| SL_Neisseria | 1854 |
| SL_Morococcus | 107 |
| SL _Kingella | 15 |

At step 3, the percent normalized abundances values of various microbial taxonomic groups are calculated individually for each of the saliva sample and the dental plaque sample, using the corresponding raw abundances mentioned in Table 6. Table 7 shows the percent normalized abundances values of various microbial taxonomic groups present in the collected samples.

Table 7

| Microbial taxonomic groups | Percent normalized abundances values |
|---|---|
| PQ_Neisseria | 1.11813 |
| PQ_Morococcus | 0.214436 |

(continued)

| Microbial taxonomic groups | Percent normalized abundances values |
|---|---|
| PQ_Kingella | 0.459506 |
| PQ_Eikenella | 0.053609 |
| ... | .... |
| ... | .... |
| SL_Neisseria | 18.5459 |
| SL_Morococcus | 1.07919 |
| SL_Kingella | 0.150666 |
| SL_Eikenella | 0.007008 |

At step 4, From the normalized abundance table, abundances of only the subset of microbial taxonomic groups which overlap with the list of two microbial taxonomic groups that are provided against the Single best training model are retained. Table 8 shows the model characteristics of features in the single best training model.

Table 8

| | SL_Mogiba cterium | PQ_Dia lister | SL_Actin omyces | PQ_Tan nerella | SL_Soloba cterium | SL_Eubac terium |
|---|---|---|---|---|---|---|
| *Q1* | 0 | 0.00182 | 0.777965 | 0.11721 4 | 0.008376 | 0.005911 |
| *Q3* | 0.02678 | 0.0997 | 2.103827 | 0.64468 8 | 0.078859 | 0.093459 |
| $Q2_4$ | 0.028023 | 0.12677 | 1.95256 | 0.64584 4 | 0.071636 | 0.093373 |
| $Q2_B$ | 0.00147 | 0.00268 | 0.890287 | 0.18216 3 | 0.010563 | 0.00832 |
| Min Model Score | 0.170987 | | | | | |
| Max Model Score | 0.495151 | | | | | |
| Thresh old | 0.264753 | | | | | |
| Numera tor/ Denomi nator | Denominat or | Denomi nator | Denomina tor | Denomin ator | Denominat or | Denomina tor |
| Model Type | Reverse | | | | | |

As an example, assume that the three taxa in the taxonomic abundance profile obtained by processing the hybrid (Saliva, Plaque) sample combination (in the manner mentioned in Steps 1 and 2) had the following rarefied abundances:

Abundance of SL_Actinomyces (i.e., feature 3 in training model) in collected saliva sample: 1.37
Abundance of PQ_Tannerella (i.e., feature 4 in training model) in collected dental plaque sample: 0.03

At step 5, Using Q1 and *Q3* values corresponding to each training model feature in the single best training model, and the transformation is applied to the above rarefied abundances. Following are the calculated transformed abundancies:

Transformed abundance (Fg_SL_Actinomyces): 0.357952
Transformed abundance (Fg_ PQ_Tannerella): 0.0

The transformed abundance of individual features as obtained above are then used appropriately in the candidate model equation ($CM_K$) (as replicated below), and numerator and denominator sums are computed. In this case, the values obtained are as follows -
Since Numerator sum = 0 and Denominator sum = 1.357952 in this case, a value of 1 is added to both numerator and denominator as per the rules:

$$CM_K = \frac{\sum F_{numerator}}{\sum F_{denominator}} \ldots \text{ when } F_{numerator} > 0 \text{ and } F_{denominator} > 0$$

or,

$$CM_K = \frac{\sum F_{numerator}+1}{\sum F_{denominator}+1} \ldots \text{ when either } F_{numerator} \text{ or } F_{denominator} = 0$$

Numerator sum: 1.000
Denominator sum: 1.357952

At step 6, the sample model score (*MS*) is computed using above Numerator sum and Denominator sum. The sample model score (*MS*) is then transformed into scaled model score (*SMS*) (having values between -1 and +1, using following rules-

$$SMS = (MS - T_{max})/(\ CMS_{K_{max}} - T_{max}),\ \ldots.. \text{ when } MS >= T_{max},$$

and

$$SMS = (MS - T_{max})/(\ T_{max} - CMS_{K_{min}}),\ \ldots.. \text{ when } MS < T_{max},$$

Wherein, $T_{max}$, $CMS_{K_{max}}$, and $CMS_{K_{min}}$ values corresponding to the respective model is used. For this purpose, the values of threshold: 0.264753, a maximum model score: 0.495151, a minimum model score: 0.170987 for single best model (as mentioned in Table 8) are employed.

Model score (*MS*): 0.736403
Scaled model score *(SMS): 0.495151*

At step 7, the *SMS* is then used for predicting the risk of ASD of the individual from whom the saliva sample and the dental plaque samples are obtained. Since both forward model and reverse model are evaluated, the final selected model is then used for classification or prediction). Here in this case, final selected single best model is a reverse model, hence the final prediction score value is calculated as (*SMS* * -1)

Final pred_score is -0.495151
Since the value is <0, the prediction class is "A" i.e., "Low risk or healthy category" Following the same series of steps, if the value of *SMS* is greater than '0' then prediction class will be "B" and thus the risk category for the subject will be "High risk of ASD".

At step 8, similarly, for the ensemble ML model, all the steps are repeated for all the single models in the ensemble and finally the average of all the final prediction score is calculated using sample model scores (*SMS*) and the class prediction is done based on final average prediction score obtained for that sample.

The written description describes the subject matter herein to enable any person skilled in the art to make and use the embodiments.

The embodiments of present disclosure herein addresses unresolved problem for accurate identification and selection of subjects with increased risk of the ASD and providing a personalized microbial cocktail for treating the ASD, using oral microbiome of the ASD affected subjects. The present disclosure attempts to offer an early, easy and reliable risk assessment and a method for design of personalized recommendable composition for applications towards amelioration of ASD.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A method (200) for risk assessment of autism spectrum disorder in a subject, comprising the steps of:

   extracting microbial deoxyribonucleic acid (DNA) sequences from each of a saliva sample and a dental plaque sample, individually, wherein the saliva sample and the dental plaque sample are received from the subject whose risk of autism spectrum disorder is to be assessed (204);
   determining a quantitative abundance of: (i) each of a plurality of predetermined microbes associated with the saliva sample and (ii) each of a plurality of predetermined microbes associated with the dental plaque sample, individually, from respective extracted DNA sequences, using a first set of probes and a second set of probes specific to each of the plurality of predetermined microbes associated with the saliva sample and the dental plaque sample respectively, through a multiplexed quantitative Polymerase Chain Reaction (qPCR) technique, wherein (i) the plurality of predetermined microbes associated with the saliva sample comprises of *Mogibacterium, Peptostreptococcus, Eubacterium, Solobacterium, Actinomyces,* and *Alistipes,* and (ii) the plurality of predetermined microbes associated with the dental plaque sample comprises of *Eubacterium, Dialister, Atopobium, Enterococcus, Mogibacterium,* and *Anaeroglobus (206);*
   collating, via one or more hardware processors, the quantitative abundance of: (i) each of the plurality of predetermined microbes associated with the saliva sample and (ii) each of the plurality of predetermined microbes associated with the dental plaque sample, to obtain a hybrid abundance matrix (208);
   determining, via the one or more hardware processors, a model score based on the hybrid abundance matrix, using a pre-determined machine learning (ML) model (210); and
   performing, via the one or more hardware processors, risk assessment of autism spectrum disorder of the subject, based on the model score and a predefined threshold value (212).

2. The method (200) as claimed in claim 1, wherein the first set of probes specific to each of the plurality of predetermined microbes associated with the saliva sample are utilized in a first multiplexed qPCR run, and a second multiplexed qPCR run, to determine the quantitative abundance of each of the plurality of predetermined microbes associated with the saliva sample, and wherein:

   (i) the plurality of predetermined microbes, the quantitative abundance of which are being determined through the first multiplexed qPCR run are: *Mogibacterium, Peptostreptococcus, Eubacterium,* and *Solobacterium;* and
   (ii) the plurality of predetermined microbes, the quantitative abundance of which are being determined through the second multiplexed qPCR run are: *Mogibacterium, Peptostreptococcus, Actinomyces,* and *Alistipes.*

3. The method (200) as claimed in claim 1, wherein the second set of probes specific to each of the plurality of predetermined microbes associated with the dental plaque sample are utilized in a third multiplexed qPCR run, and a fourth multiplexed qPCR run, to determine the quantitative abundance of each of the plurality of predetermined microbes associated with the dental plaque sample, and wherein:

(i) the plurality of predetermined microbes, the quantitative abundance of which are being determined through the third multiplexed qPCR run are: *Eubacterium, Dialister, Atopobium,* and *Enterococcus;* and
(ii) the plurality of predetermined microbes, the quantitative abundance of which are being determined through the fourth multiplexed qPCR run are: *Eubacterium, Dialister, Mogibacterium,* and *Anaeroglobus.*

4. The method (200) as claimed in claim 1, wherein the pre-determined machine learning (ML) model is an ensemble ML model that is built using a microbial abundance data corresponding to a plurality of training saliva samples and a plurality of training dental plaque samples.

5. The method (200) as claimed in claim 1, wherein the plurality of predetermined microbes associated with the saliva sample and the plurality of predetermined microbes associated with the dental plaque sample are features of the pre-determined machine learning (ML) model.

6. The method (200) as claimed in claim 1, wherein the first set of probes specific to each of the plurality of predetermined microbes associated with the saliva sample are utilized in a first multiplexed qPCR run, and a second multiplexed qPCR run, to determine the quantitative abundance of each of the plurality of predetermined microbes associated with the saliva sample, wherein one or more predetermined microbes out of the plurality of predetermined microbes associated with the saliva sample, are common to the first multiplexed qPCR run and the second multiplexed qPCR run for determining the quantitative abundance, and wherein the one or more predetermined microbes that are common to the first multiplexed qPCR run and the second multiplexed qPCR run are determined based on (i) a median abundance of each of the plurality of predetermined microbes obtained from the plurality of training saliva samples, (ii) a frequency of occurrence of each of the plurality of predetermined microbes constituting the ensemble ML model.

7. The method as claimed in claim 1, wherein the second set of probes specific to each of the plurality of predetermined microbes associated with the dental plaque sample are utilized in a third multiplexed qPCR run, and a fourth multiplexed qPCR run, to determine the quantitative abundance of each of the plurality of predetermined microbes associated with the dental plaque sample, wherein one or more predetermined microbes out of the plurality of predetermined microbes associated with the dental plaque sample are common to the third multiplexed qPCR run and the fourth multiplexed qPCR run for determining the quantitative abundance, and wherein the one or more predetermined microbes that are common to the third multiplexed qPCR run and the fourth multiplexed qPCR run are determined based on (i) a median abundance of each of the plurality of predetermined microbes obtained from the plurality of training dental plaque samples, (ii) a frequency of occurrence of each of the plurality of predetermined microbes constituting the ensemble ML model.

**Patentansprüche**

1. Verfahren (200) zur Risikobewertung einer Autismus-Spektrum-Störung bei einem Subjekt, umfassend die Schritte:

Extrahieren von mikrobiellen Desoxyribonukleinsäure(DNA)-Sequenzen aus jeweils einer Speichelprobe und einer Zahnbelagprobe, wobei die Speichelprobe und die Zahnbelagprobe von dem Subjekt erhalten werden, dessen Risiko einer Autismus-Spektrum-Störung zu bewerten ist (204);
Bestimmen einer quantitativen Häufigkeit von: (i) jeder einer Mehrzahl von vorbestimmten Mikroben, die mit der Speichelprobe assoziiert sind, und (ii) jeder einer Mehrzahl von vorbestimmten Mikroben, die mit der Zahnbelagprobe assoziiert sind, aus jeweiligen extrahierten DNA-Sequenzen unter Verwendung eines ersten Satzes von Sonden und eines zweiten Satzes von Sonden, die für jede der Mehrzahl von vorbestimmten Mikroben spezifisch sind, die jeweils mit der Speichelprobe und der Zahnbelagprobe assoziiert sind, durch eine gemultiplexte quantitative Polymerasekettenreaktion(quantitative Polymerase Chain Reaction, qPCR)-Technik, wobei (i) die Mehrzahl von vorbestimmten Mikroben, die mit der Speichelprobe assoziiert sind, *Mogibacterium, Peptostreptococcus, Eubacterium, Solobacterium, Actinomyces* und *Alistipes* umfasst, und (ii) die Mehrzahl von vorbestimmten Mikroben, die mit der Zahnbelagprobe assoziiert sind, *Eubacterium, Dialister, Atopobium,*

*Enterococcus, Mogibacterium* und *Anaeroglobus* umfasst (206);
Zusammenstellen, über einen oder mehrere Hardwareprozessoren, der quantitativen Häufigkeit von: (i) jeder der Mehrzahl von vorbestimmten Mikroben, die mit der Speichelprobe assoziiert sind, und (ii) jeder der Mehrzahl von vorbestimmten Mikroben, die mit der Zahnbelagprobe assoziiert sind, um eine hybride Häufigkeitsmatrix zu erhalten (208);
Bestimmen, über den einen oder die mehreren Hardwareprozessoren, einer Modellbewertung basierend auf der hybriden Häufigkeitsmatrix unter Verwendung eines vorbestimmten Maschinenlern(ML)-Modells (210); und
Durchführen, über den einen oder die mehreren Hardwareprozessoren, einer Risikobewertung einer Autismus-Spektrum-Störung des Subjekts basierend auf der Modellbewertung und einem vordefinierten Schwellenwert (212).

**2.** Verfahren (200) nach Anspruch 1, wobei der erste Satz von Sonden, die für jede der Mehrzahl von vorbestimmten Mikroben spezifisch sind, die mit der Speichelprobe assoziiert sind, in einem ersten gemultiplexten qPCR-Durchlauf und einem zweiten gemultiplexten qPCR-Durchlauf verwendet wird, um die quantitative Häufigkeit von jeder der Mehrzahl von vorbestimmten Mikroben zu bestimmen, die mit der Speichelprobe assoziiert sind, und wobei:

(i) die Mehrzahl von vorbestimmten Mikroben, deren quantitative Häufigkeit durch den ersten gemultiplexten qPCR-Durchlauf bestimmt wird, Folgendes sind: *Mogibacterium, Peptostreptococcus, Eubacterium* und *Solobacterium;* und
(ii) die Mehrzahl von vorbestimmten Mikroben, deren quantitative Häufigkeit durch den zweiten gemultiplexten qPCR-Durchlauf bestimmt wird, Folgendes sind: *Mogibacterium, Peptostreptococcus, Actinomyces* und *Alistipes.*

**3.** Verfahren (200) nach Anspruch 1, wobei der zweite Satz von Sonden, die für jede der Mehrzahl von vorbestimmten Mikroben spezifisch sind, die mit der Zahnbelagprobe assoziiert sind, in einem dritten gemultiplexten qPCR-Durchlauf und einem vierten gemultiplexten qPCR-Durchlauf verwendet wird, um die quantitative Häufigkeit von jeder der Mehrzahl von vorbestimmten Mikroben zu bestimmen, die mit der Zahnbelagprobe assoziiert sind, und wobei:

(i) die Mehrzahl von vorbestimmten Mikroben, deren quantitative Häufigkeit durch den dritten gemultiplexten qPCR-Durchlauf bestimmt wird, Folgendes sind: *Eubacterium, Dialister, Atopobium* und *Enterococcus;* und
(ii) die Mehrzahl von vorbestimmten Mikroben, deren quantitative Häufigkeit durch den vierten gemultiplexten qPCR-Durchlauf bestimmt wird, Folgendes sind: *Eubacterium, Dialister, Mogibacterium und Anaeroglobus.*

**4.** Verfahren (200) nach Anspruch 1, wobei das vorbestimmte Maschinenlern(ML)-Modell ein Ensemble-ML-Modell ist, das unter Verwendung von Mikrobenhäufigkeitsdaten erstellt wird, die einer Mehrzahl von Trainingsspeichelproben und einer Mehrzahl von Trainingszahnbelagproben entsprechen.

**5.** Verfahren (200) nach Anspruch 1, wobei die Mehrzahl von vorbestimmten Mikroben, die mit der Speichelprobe assoziiert sind, und die Mehrzahl von vorbestimmten Mikroben, die mit der Zahnbelagprobe assoziiert sind, Merkmale des vorbestimmten Maschinenlern(ML)-Modells sind.

**6.** Verfahren (200) nach Anspruch 1, wobei der erste Satz von Sonden, die für jede der Mehrzahl von vorbestimmten Mikroben spezifisch sind, die mit der Speichelprobe assoziiert sind, in einem ersten gemultiplexten qPCR-Durchlauf und einem zweiten gemultiplexten qPCR-Durchlauf verwendet wird, um die quantitative Häufigkeit von jeder der Mehrzahl von vorbestimmten Mikroben zu bestimmen, die mit der Speichelprobe assoziiert sind, wobei ein oder mehrere vorbestimmte Mikroben aus der Mehrzahl von vorbestimmten Mikroben, die mit der Speichelprobe assoziiert sind, dem ersten gemultiplexten qPCR-Durchlauf und dem zweiten gemultiplexten qPCR-Durchlauf gemeinsam sind, um die quantitative Häufigkeit zu bestimmen, und wobei das eine oder die mehreren vorbestimmten Mikroben, die dem ersten gemultiplexten qPCR-Durchlauf und dem zweiten gemultiplexten qPCR-Durchlauf gemeinsam sind, basierend auf (i) einer mittleren Häufigkeit von jeder der Mehrzahl von vorbestimmten Mikroben, die aus der Mehrzahl von Trainingsspeichelproben erhalten werden, (ii) einer Häufigkeit des Auftretens von jeder der Mehrzahl von vorbestimmten Mikroben, die das Ensemble-ML-Modell bilden, bestimmt werden.

**7.** Verfahren nach Anspruch 1, wobei der zweite Satz von Sonden, die für jede der Mehrzahl von vorbestimmten Mikroben spezifisch sind, die mit der Zahnbelagprobe assoziiert sind, in einem dritten gemultiplexten qPCR-Durchlauf und einem vierten gemultiplexten qPCR-Durchlauf verwendet wird, um die quantitative Häufigkeit von jeder der Mehrzahl von vorbestimmten Mikroben zu bestimmen, die mit der Zahnbelagprobe assoziiert sind, wobei ein oder mehrere vorbestimmte Mikroben aus der Mehrzahl von vorbestimmten Mikroben, die mit der Zahnbelagprobe assoziiert sind, dem dritten gemultiplexten qPCR-Durchlauf und dem vierten gemultiplexten qPCR-Durchlauf gemeinsam sind, um die quantitative Häufigkeit zu bestimmen, und wobei das eine oder die mehreren vorbestimmten Mikroben, die dem dritten gemultiplexten qPCR-Durchlauf und dem vierten gemultiplexten qPCR-Durchlauf gemeinsam sind, basierend auf (i) einer mittleren Häufigkeit von jeder der Mehrzahl von vorbestimmten Mikroben, die aus der Mehrzahl von Trainingszahnbelagproben erhalten werden, (ii) einer Häufigkeit des Auftretens von jeder der Mehrzahl von vorbestimmten Mikroben, die das Ensemble-ML-Modell bilden, bestimmt werden.

## Revendications

1. Procédé (200) d'évaluation du risque de trouble du spectre de l'autisme chez un sujet, comprenant les étapes consistant à :

   extraire des séquences d'acide désoxyribonucléique (ADN) microbiennes de chacun d'un échantillon de salive et d'un échantillon de plaque dentaire, individuellement, dans lequel l'échantillon de salive et l'échantillon de plaque dentaire sont reçus du sujet dont le risque de trouble du spectre de l'autisme doit être évalué (204) ;
   déterminer une abondance quantitative de : (i) chacun d'une pluralité de microbes prédéterminés associés à l'échantillon de salive et (ii) chacun d'une pluralité de microbes prédéterminés associés à l'échantillon de plaque dentaire, individuellement, à partir de séquences d'ADN extraites respectives, en utilisant un premier ensemble de sondes et un second ensemble de sondes spécifiques à chacun de la pluralité de microbes prédéterminés associés à l'échantillon de salive et à l'échantillon de plaque dentaire, respectivement, par l'intermédiaire d'une technique de réaction en chaîne par polymérase quantitative multiplexée (qPCR), dans lequel (i) la pluralité de microbes prédéterminés associés à l'échantillon de salive comprend *Mogibacterium, Peptostreptococcus, Eubacterium, Solobacterium, Actinomyces* et *Alistipes,* et (ii) la pluralité de microbes prédéterminés associés à l'échantillon de plaque dentaire comprend *Eubacterium, Dialister, Atopobium, Enterococcus, Mogibacterium* et *Anaeroglobus* (206) ;
   rassembler, par l'intermédiaire d'un ou plusieurs processeurs matériels, l'abondance quantitative de : (i) chacun de la pluralité de microbes prédéterminés associés à l'échantillon de salive et (ii) chacun de la pluralité de microbes prédéterminés associés à l'échantillon de plaque dentaire, pour obtenir une matrice d'abondance hybride (208) ;
   déterminer, par l'intermédiaire des un ou plusieurs processeurs matériels, un score de modèle sur la base de la matrice d'abondance hybride, en utilisant un modèle d'apprentissage machine (ML) prédéterminé (210) ; et
   effectuer, par l'intermédiaire des un ou plusieurs processeurs matériels, une évaluation du risque de trouble du spectre de l'autisme du sujet, sur la base du score de modèle et d'une valeur de seuil prédéfinie (212).

2. Procédé (200) selon la revendication 1, dans lequel le premier ensemble de sondes spécifiques à chacun de la pluralité de microbes prédéterminés associés à l'échantillon de salive sont utilisées dans un premier cycle de qPCR multiplexée, et un deuxième cycle de qPCR multiplexée, pour déterminer l'abondance quantitative de chacun de la pluralité de microbes prédéterminés associés à l'échantillon de salive, et dans lequel :

   (i) la pluralité de microbes prédéterminés, dont l'abondance quantitative est déterminée par l'intermédiaire du premier cycle de qPCR multiplexée sont *: Mogibacterium, Peptostreptococcus, Eubacterium* et *Solobacterium ;* et
   (ii) la pluralité de microbes prédéterminés, dont l'abondance quantitative est déterminée par l'intermédiaire du deuxième cycle de qPCR multiplexée sont *: Mogibacterium, Peptostreptococcus, Actinomyces* et *Alistipes.*

3. Procédé (200) selon la revendication 1, dans lequel le second ensemble de sondes spécifiques à chacun de la pluralité de microbes prédéterminés associés à l'échantillon de plaque dentaire sont utilisées dans un troisième cycle de qPCR multiplexée, et un quatrième cycle de qPCR multiplexée, pour déterminer l'abondance quantitative de chacun de la pluralité de microbes prédéterminés associés à l'échantillon de plaque dentaire, et dans lequel :

   (i) la pluralité de microbes prédéterminés, dont l'abondance quantitative est déterminée par l'intermédiaire du troisième cycle de qPCR multiplexée sont *: Eubacterium, Dialister, Atopobium* et *Enterococcus ;* et
   (ii) la pluralité de microbes prédéterminés, dont l'abondance quantitative est déterminée par l'intermédiaire du quatrième cycle de qPCR multiplexée *sont : Eubacterium, Dialister, Mogibacterium* et Anaeroglobus.

4. Procédé (200) selon la revendication 1, dans lequel le modèle d'apprentissage machine (ML) prédéterminé est un modèle ML ensembliste qui est construit en utilisant des données d'abondance microbienne correspondant à une pluralité d'échantillons de salive d'apprentissage et une pluralité d'échantillons de plaque dentaire d'apprentissage.

**5.** Procédé (200) selon la revendication 1, dans lequel la pluralité de microbes prédéterminés associés à l'échantillon de salive et la pluralité de microbes prédéterminés associés à l'échantillon de plaque dentaire sont des caractéristiques du modèle d'apprentissage machine (ML) prédéterminé.

**6.** Procédé (200) selon la revendication 1, dans lequel le premier ensemble de sondes spécifiques à chacun de la pluralité de microbes prédéterminés associés à l'échantillon de salive sont utilisées dans un premier cycle de qPCR multiplexée, et un deuxième cycle de qPCR multiplexée, pour déterminer l'abondance quantitative de chacun de la pluralité de microbes prédéterminés associés à l'échantillon de salive, dans lequel un ou plusieurs microbes prédéterminés parmi la pluralité de microbes prédéterminés associés à l'échantillon de salive, sont communs au premier cycle de qPCR multiplexée et au deuxième cycle de qPCR multiplexée pour déterminer l'abondance quantitative, et dans lequel les un ou plusieurs microbes prédéterminés qui sont communs au premier cycle de qPCR multiplexée et au deuxième cycle de qPCR multiplexée sont déterminés sur la base (i) d'une abondance médiane de chacun de la pluralité de microbes prédéterminés obtenus à partir de la pluralité d'échantillons de salive d'apprentissage, (ii) d'une fréquence d'occurrence de chacun de la pluralité de microbes prédéterminés constituant le modèle ML ensembliste.

**7.** Procédé selon la revendication 1, dans lequel le second ensemble de sondes spécifiques à chacun de la pluralité de microbes prédéterminés associés à l'échantillon de plaque dentaire sont utilisées dans un troisième cycle de qPCR multiplexée, et un quatrième cycle de qPCR multiplexée, pour déterminer l'abondance quantitative de chacun de la pluralité de microbes prédéterminés associés à l'échantillon de plaque dentaire, dans lequel un ou plusieurs microbes prédéterminés parmi la pluralité de microbes prédéterminés associés à l'échantillon de plaque dentaire sont communs au troisième cycle de qPCR multiplexée et au quatrième cycle de qPCR multiplexée pour déterminer l'abondance quantitative, et dans lequel les un ou plusieurs microbes prédéterminés qui sont communs au troisième cycle de qPCR multiplexée et au quatrième cycle de qPCR multiplexée sont déterminés sur la base (i) d'une abondance médiane de chacun de la pluralité de microbes prédéterminés obtenus à partir de la pluralité d'échantillons de plaque dentaire d'apprentissage, (ii) d'une fréquence d'occurrence de chacun de la pluralité de microbes prédéterminés constituant le modèle ML ensembliste.

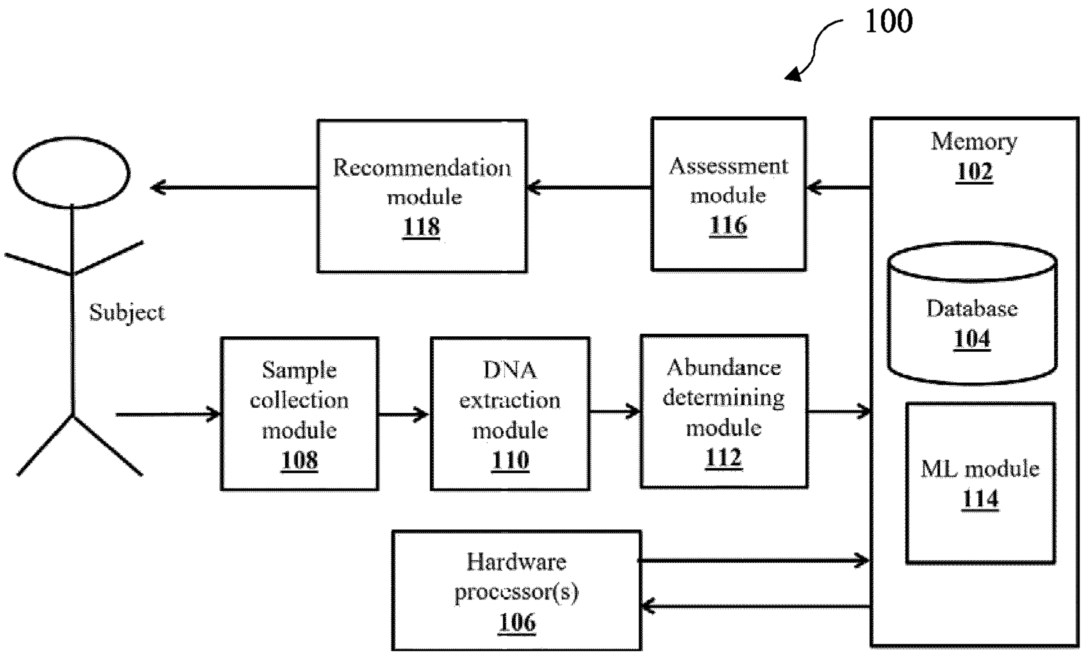
100
Recommendation module 118
Assessment module 116
Memory 102
Database 104
Subject
Sample collection module 108
DNA extraction module 110
Abundance determining module 112
ML module 114
Hardware processor(s) 106

FIG. 1

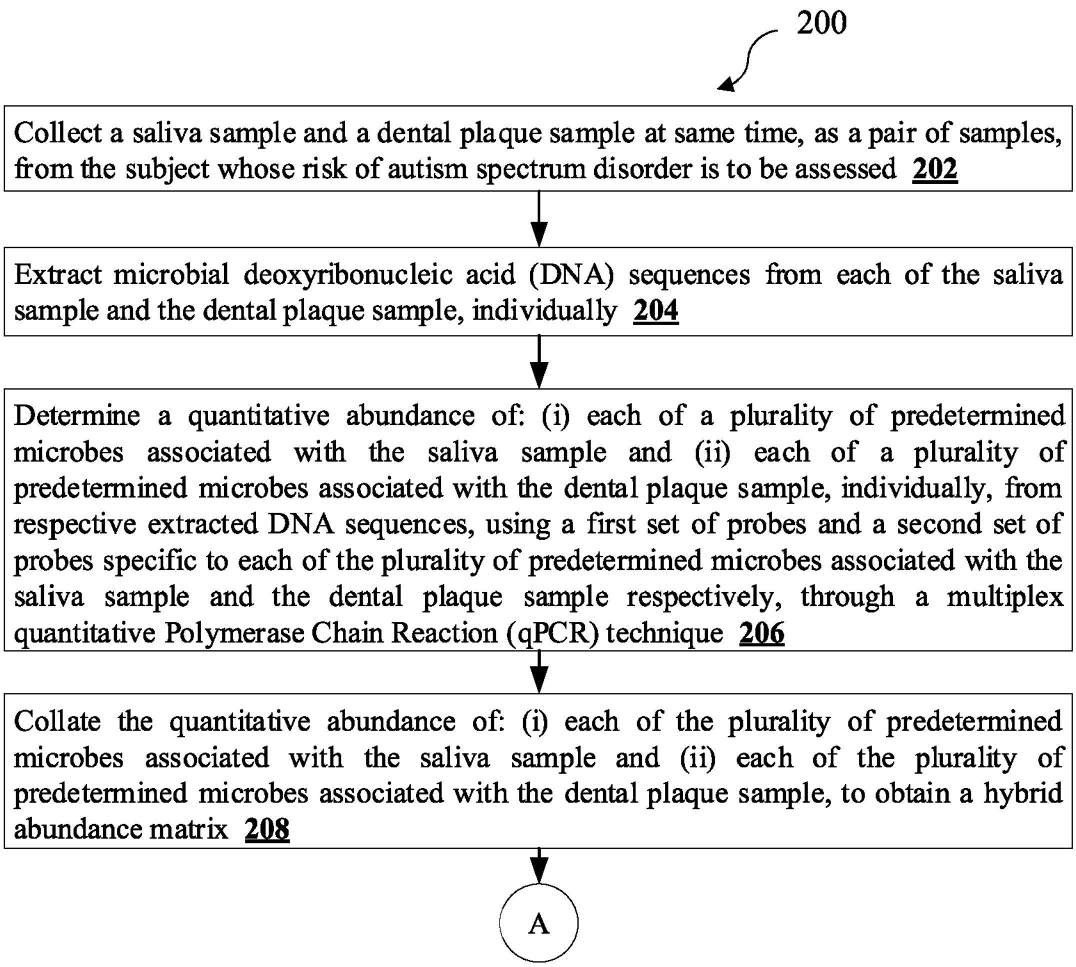
200
Collect a saliva sample and a dental plaque sample at same time, as a pair of samples, from the subject whose risk of autism spectrum disorder is to be assessed 202
Extract microbial deoxyribonucleic acid (DNA) sequences from each of the saliva sample and the dental plaque sample, individually 204
Determine a quantitative abundance of: (i) each of a plurality of predetermined microbes associated with the saliva sample and (ii) each of a plurality of predetermined microbes associated with the dental plaque sample, individually, from respective extracted DNA sequences, using a first set of probes and a second set of probes specific to each of the plurality of predetermined microbes associated with the saliva sample and the dental plaque sample respectively, through a multiplex quantitative Polymerase Chain Reaction (qPCR) technique 206
Collate the quantitative abundance of: (i) each of the plurality of predetermined microbes associated with the saliva sample and (ii) each of the plurality of predetermined microbes associated with the dental plaque sample, to obtain a hybrid abundance matrix 208
A

FIG. 2A

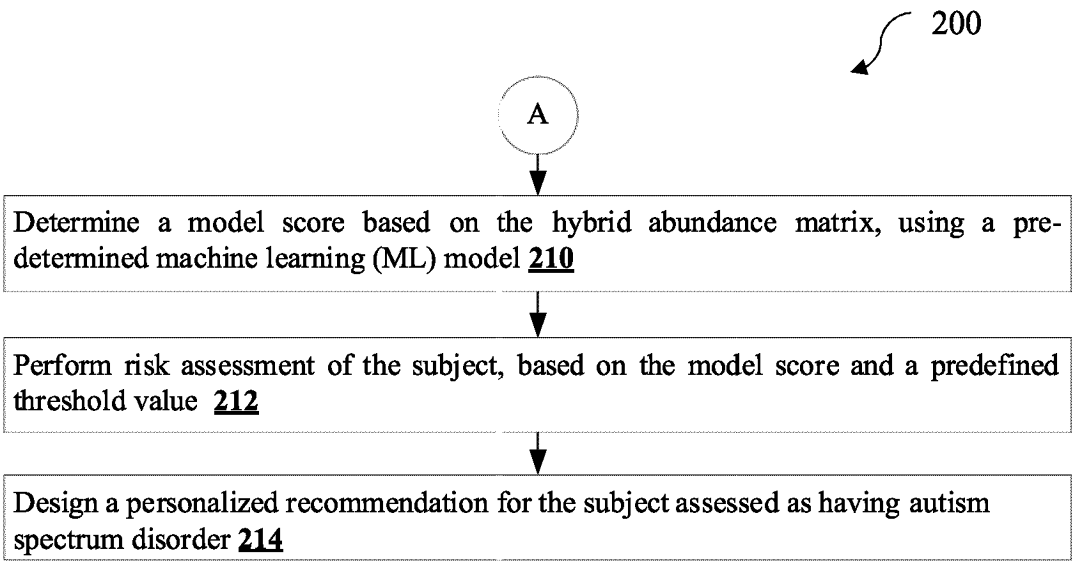
200
A
Determine a model score based on the hybrid abundance matrix, using a pre-determined machine learning (ML) model 210
Perform risk assessment of the subject, based on the model score and a predefined threshold value 212
Design a personalized recommendation for the subject assessed as having autism spectrum disorder 214

FIG. 2B

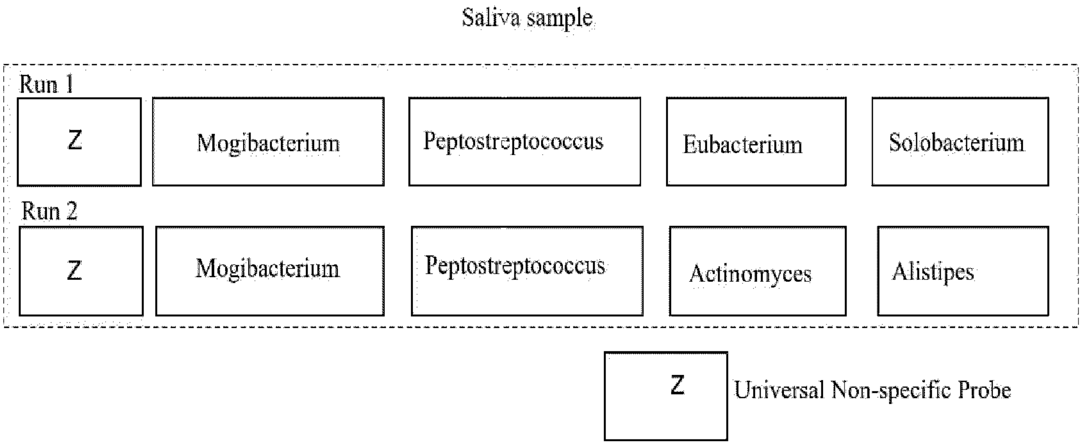
Saliva sample
Run 1
Z
Mogibacterium
Peptostreptococcus
Eubacterium
Solobacterium
Run 2
Z
Mogibacterium
Peptostreptococcus
Actinomyces
Alistipes
Z
Universal Non-specific Probe

FIG. 3A

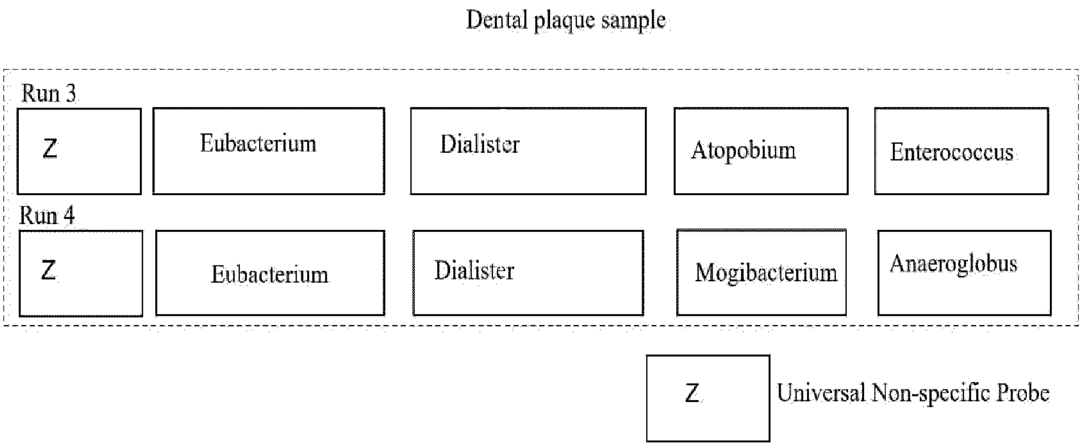
Dental plaque sample
Run 3
Z
Eubacterium
Dialister
Atopobium
Enterococcus
Run 4
Z
Eubacterium
Dialister
Mogibacterium
Anaeroglobus
Z
Universal Non-specific Probe

FIG. 3B

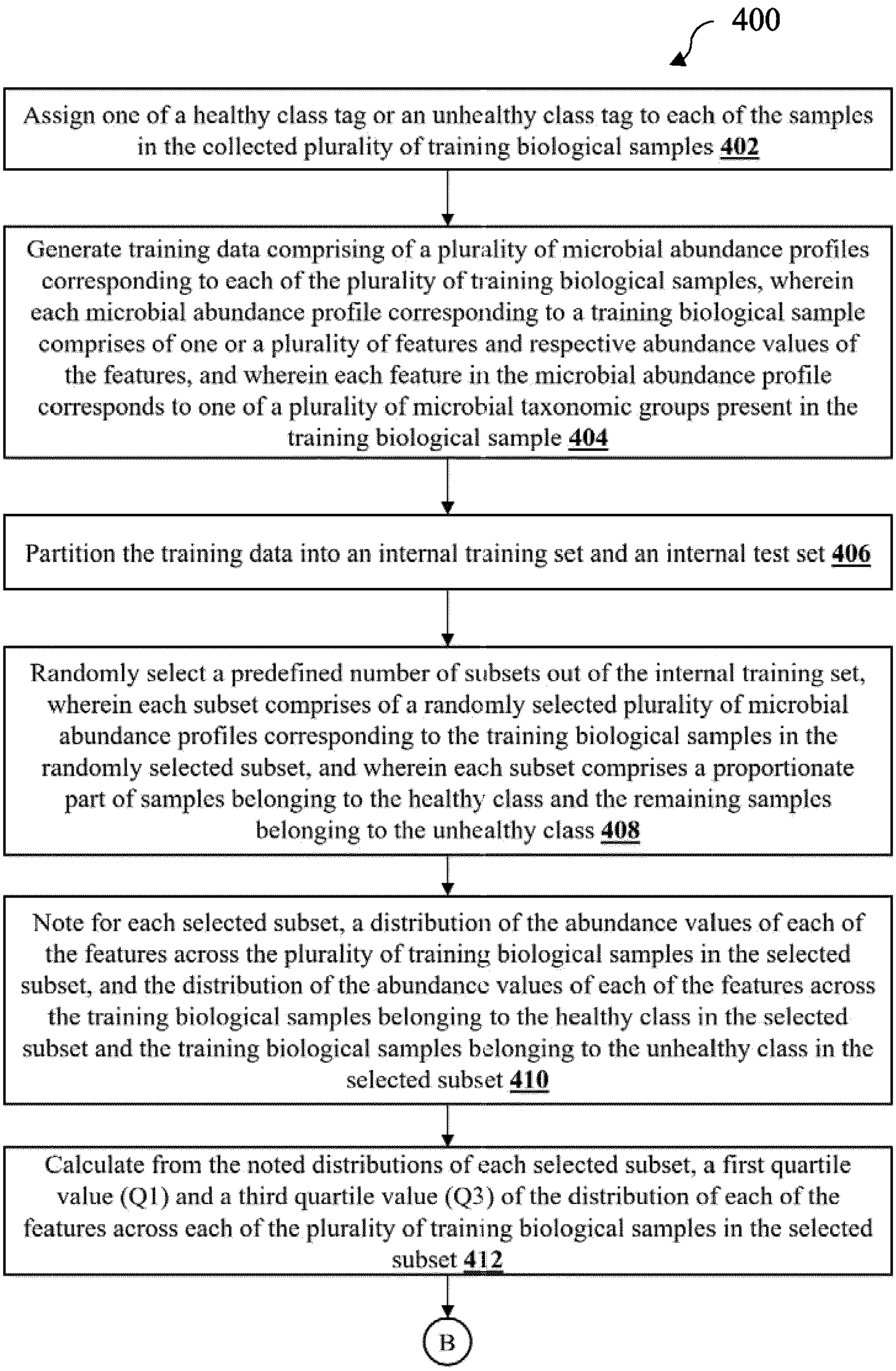
400
Assign one of a healthy class tag or an unhealthy class tag to each of the samples in the collected plurality of training biological samples 402
Generate training data comprising of a plurality of microbial abundance profiles corresponding to each of the plurality of training biological samples, wherein each microbial abundance profile corresponding to a training biological sample comprises of one or a plurality of features and respective abundance values of the features, and wherein each feature in the microbial abundance profile corresponds to one of a plurality of microbial taxonomic groups present in the training biological sample 404
Partition the training data into an internal training set and an internal test set 406
Randomly select a predefined number of subsets out of the internal training set, wherein each subset comprises of a randomly selected plurality of microbial abundance profiles corresponding to the training biological samples in the randomly selected subset, and wherein each subset comprises a proportionate part of samples belonging to the healthy class and the remaining samples belonging to the unhealthy class 408
Note for each selected subset, a distribution of the abundance values of each of the features across the plurality of training biological samples in the selected subset, and the distribution of the abundance values of each of the features across the training biological samples belonging to the healthy class in the selected subset and the training biological samples belonging to the unhealthy class in the selected subset 410
Calculate from the noted distributions of each selected subset, a first quartile value (Q1) and a third quartile value (Q3) of the distribution of each of the features across each of the plurality of training biological samples in the selected subset 412
B

FIG. 4A

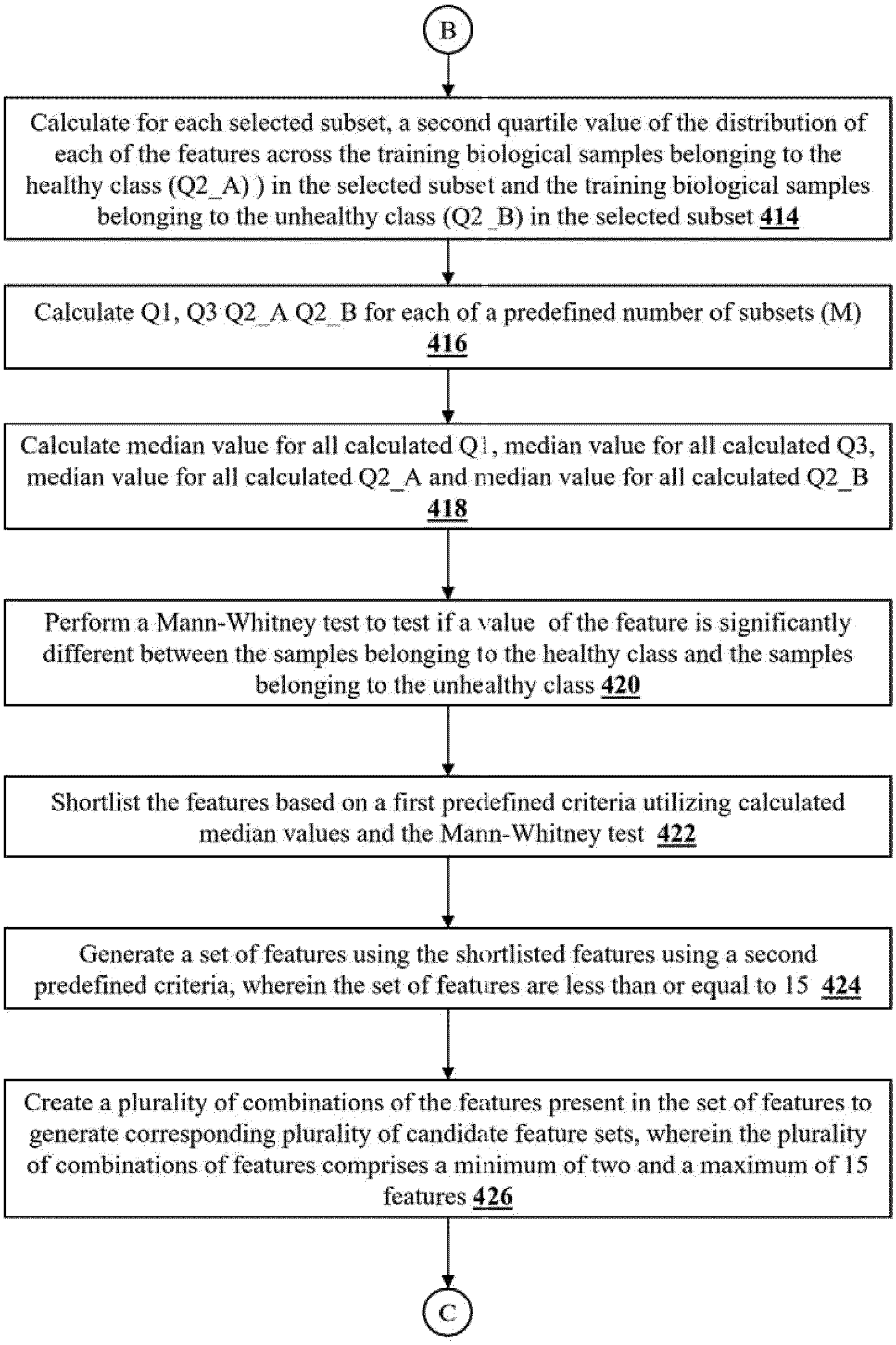
B
Calculate for each selected subset, a second quartile value of the distribution of each of the features across the training biological samples belonging to the healthy class (Q2_A) ) in the selected subset and the training biological samples belonging to the unhealthy class (Q2_B) in the selected subset 414
Calculate Q1, Q3 Q2_A Q2_B for each of a predefined number of subsets (M) 416
Calculate median value for all calculated Q1, median value for all calculated Q3, median value for all calculated Q2_A and median value for all calculated Q2_B 418
Perform a Mann-Whitney test to test if a value of the feature is significantly different between the samples belonging to the healthy class and the samples belonging to the unhealthy class 420
Shortlist the features based on a first predefined criteria utilizing calculated median values and the Mann-Whitney test 422
Generate a set of features using the shortlisted features using a second predefined criteria, wherein the set of features are less than or equal to 15 424
Create a plurality of combinations of the features present in the set of features to generate corresponding plurality of candidate feature sets, wherein the plurality of combinations of features comprises a minimum of two and a maximum of 15 features 426
C

FIG. 4B

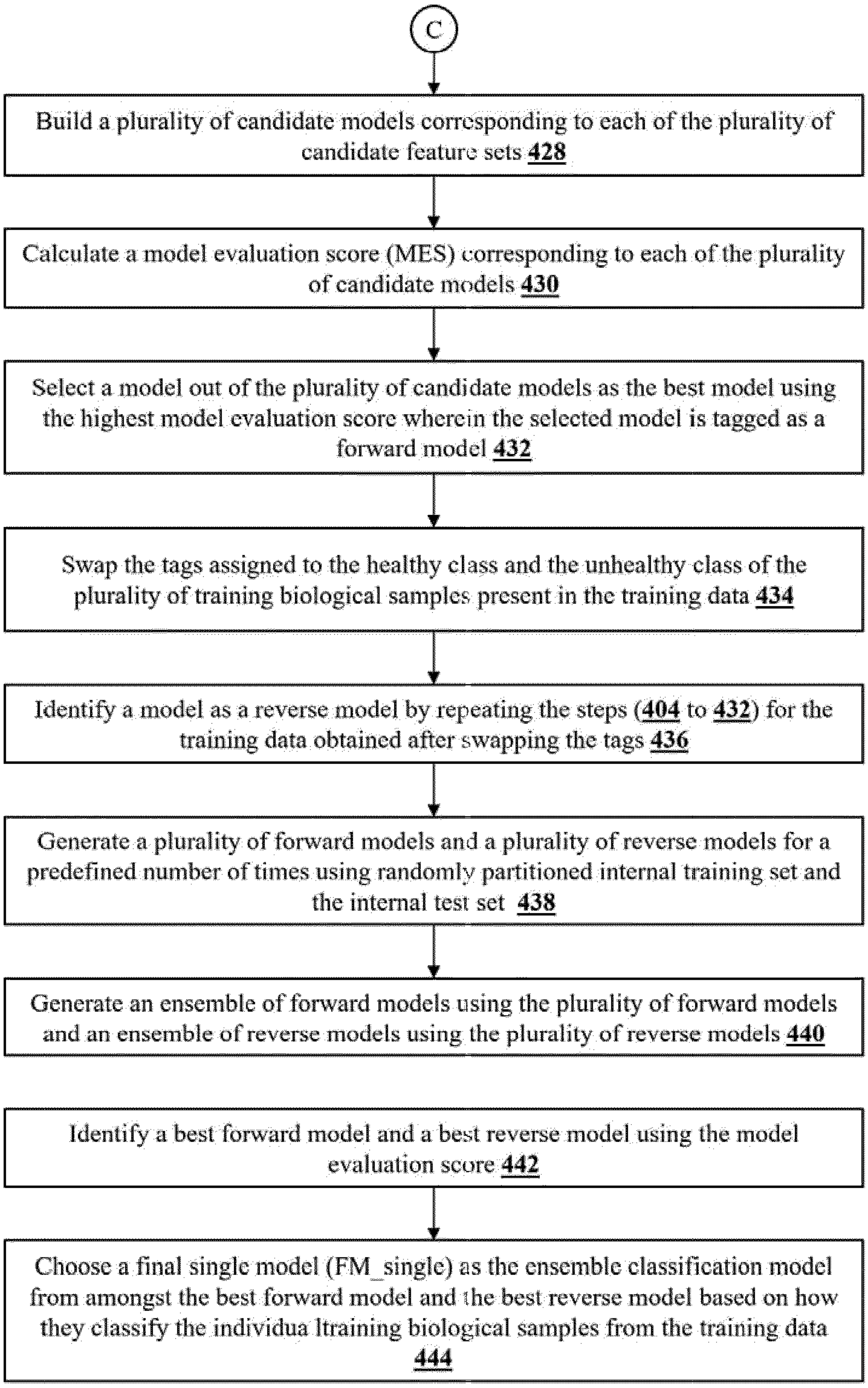
C
Build a plurality of candidate models corresponding to each of the plurality of candidate feature sets 428
Calculate a model evaluation score (MES) corresponding to each of the plurality of candidate models 430
Select a model out of the plurality of candidate models as the best model using the highest model evaluation score wherein the selected model is tagged as a forward model 432
Swap the tags assigned to the healthy class and the unhealthy class of the plurality of training biological samples present in the training data 434
Identify a model as a reverse model by repeating the steps (404 to 432) for the training data obtained after swapping the tags 436
Generate a plurality of forward models and a plurality of reverse models for a predefined number of times using randomly partitioned internal training set and the internal test set 438
Generate an ensemble of forward models using the plurality of forward models and an ensemble of reverse models using the plurality of reverse models 440
Identify a best forward model and a best reverse model using the model evaluation score 442
Choose a final single model (FM_single) as the ensemble classification model from amongst the best forward model and the best reverse model based on how they classify the individua ltraining biological samples from the training data 444

FIG. 4C

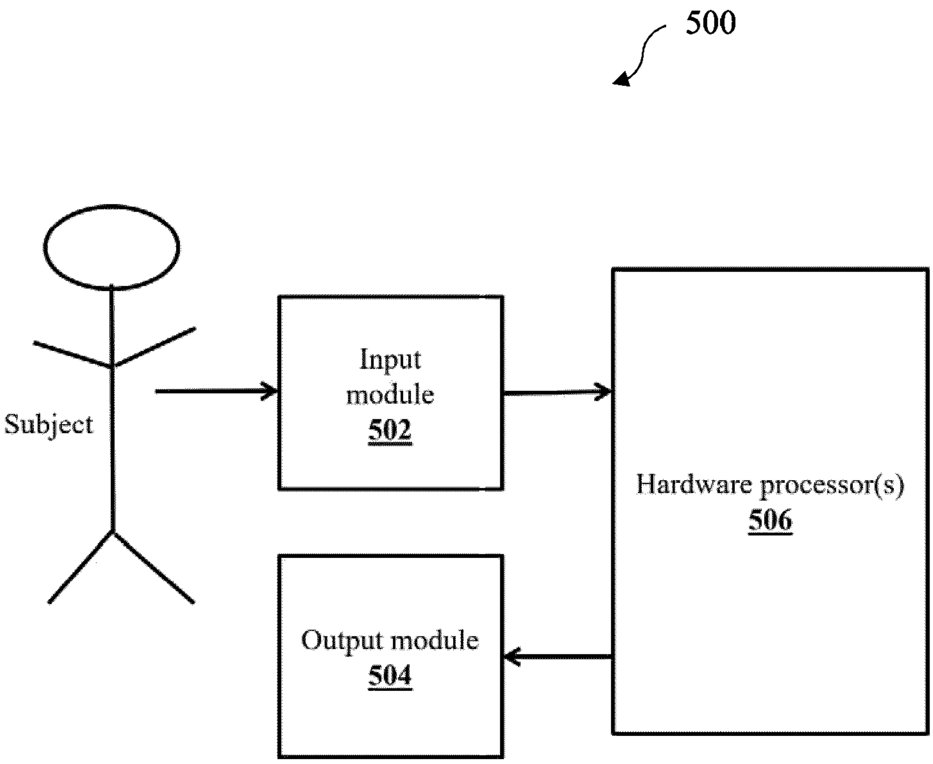
500
Subject
Input
module
502
Hardware processor(s)
506
Output module
504

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IN 202321028609

### Non-patent literature cited in the description

- **QIAO YANAN et al.** *Alterations of oral microbiota distinguish children with autism spectrum disorders from healthy controls*